(19) Europäisches Patentamt
European Patent Office
Office européen des brevets

(11) EP 1 554 016 B1

(12) **EUROPEAN PATENT SPECIFICATION**

(45) Date of publication and mention of the grant of the patent:
**14.05.2008 Bulletin 2008/20**

(21) Application number: **03757724.4**

(22) Date of filing: **15.10.2003**

(51) Int Cl.:
***A61P 15/08*** (2006.01) ***C12N 5/00*** (2006.01)

(86) International application number:
**PCT/DK2003/000703**

(87) International publication number:
**WO 2004/035766 (29.04.2004 Gazette 2004/18)**

(54) **IN VITRO SYNCHRONISATION OF NUCLEAR AND CYTOPLASMATIC MATURATION OF OOCYTES INVOLVING ADDITION AND REMOVAL OF PHOSPHODIESTERASE TYPE 3 INHIBITOR**

IN-VITRO-SYNCHRONISATION DER KERN- UND ZYTOPLASMA-REIFUNG VON OOZYTEN MIT ADDITION UND ENTFERNUNG VON PHOSPHODIESTERASE-HEMMER TYP 3

SYNCHRONISATION IN VITRO DE LA MATURATION NUCLEAIRE ET CYTOPLASMIQUE DES OOCYTES COMPRENANT L'AJOUT ET LE RETRAIT D'INHIBITEUR DE LA PHOSPHODIESTERASE DE TYPE 3

(84) Designated Contracting States:
**AT BE BG CH CY CZ DE DK EE ES FI FR GB GR HU IE IT LI LU MC NL PT RO SE SI SK TR**

(30) Priority: **15.10.2002 DK 200201581**

(43) Date of publication of application:
**20.07.2005 Bulletin 2005/29**

(73) Proprietors:
- **NOVO NORDISK A/S**
  **2880 Bagsvaerd (DK)**
- **Bayer Schering Pharma Aktiengesellschaft**
  **13353 Berlin (DE)**

(72) Inventors:
- **SMITZ, Johan**
  **B-1090 Brussels (BE)**
- **GRONDAHL, Christian**
  **DK-3500 Vaerlose (DK)**

(56) References cited:
**WO-A-01/38493** **WO-A-01/76360**
**WO-A-93/00863**

- **REBECKA E. THOMAS ET AL: "Differential Effects of Specific Phosphodiesterase Isoenzyme Inhibitors on Bovine Oocyte Meiotic Maturation" DEVELOPMENTAL BIOLOGY, vol. 244, 2002, pages 215-225, XP002268443**
- **DATABASE MEDLINE [Online] December 2000 (2000-12) MIKKELSEN A L ET AL: "Possible factors affecting the development of oocytes in in-vitro maturation." Database accession no. NLM11263532 XP002268446 & HUMAN REPRODUCTION (OXFORD, ENGLAND) ENGLAND DEC 2000, vol. 15 Suppl 5, December 2000 (2000-12), pages 11-17, ISSN: 0268-1161**
- **A. WIERSMA ET AL: "Phosphodiesterase 3 Inhibitors Suppress Oocyte Maturation and Consequent Pregnance without Affecting Ovulation and Cyclicity in Rodents" J. CLIN. INVEST., vol. 102, no. 3, August 1998 (1998-08), pages 532-537, XP002268444**
- **A. TSAFRIRI ET AL: "Oocyte Maturation Involves Compartmentalization and Opposing Changes of cAMP Levels in Follicular Somatic and Germ Cells: Studies Using Selective Phosphodiesterase Inhibitors" DEVELOPMENTAL BIOLOGY, vol. 178, 1996, pages 393-402, XP002268445**
- **DATABASE MEDLINE [Online] December 2003 (2003-12) NOGUEIRA D ET AL: "Effect of phosphodiesterase type 3 inhibitor on developmental competence of immature mouse oocytes in vitro." Database accession no. NLM12930710 XP002268447 & BIOLOGY OF REPRODUCTION. UNITED STATES DEC 2003, vol. 69, no. 6, December 2003 (2003-12), pages 2045-2052, ISSN: 0006-3363**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- DATABASE MEDLINE [Online] September 2003 (2003-09) NOGUEIRA DANIELA ET AL: "Human oocytes reversibly arrested in prophase I by phosphodiesterase type 3 inhibitor in vitro." Database accession no. NLM12773402 XP002268448 & BIOLOGY OF REPRODUCTION. UNITED STATES SEP 2003, vol. 69, no. 3, September 2003 (2003-09), pages 1042-1052, ISSN: 0006-3363

## Description

The present invention relates to a method for in vitro synchronisation of nuclear and cytoplasmatic maturation of germinal vesicle (hereinafter designated GV) oocytes from domestic animals or from primates. More particularly, the present invention relates to the aspects defined in the claims below.

BACKGROUND OF THIS INVENTION

Meiosis is the unique and ultimate event of germ cells on which sexual reproduction is based. Meiosis comprises two meiotic divisions. During the first division, exchange between maternal and paternal genes take place before the pairs of chromosomes are separated into the two daughter cells. These contain only half the number (1 n) of chromosomes and 2c DNA. The second meiotic division proceeds without a DNA synthesis. This division therefore results in the formation of the haploid germ cells with only 1c DNA.

The meiotic events are similar in the male and female germ cells, but the time schedule and the differentiation processes which lead to ova and to spermatozoa differ profoundly. All female germ cells enter the prophase of the first meiotic division early in life, often before birth, but all are arrested as oocytes later in the prophase (dictyate state) until ovulation after puberty. Thus, from early life, the female has a stock of oocytes which is drawn upon until the stock is exhausted. Meiosis in females is not completed until after fertilization, and results in only one ovum and two abortive polar bodies per germ cell. In contrast, only some of the male germ cells enter meiosis from puberty and leave a stem population of germ cells throughout life. Once initiated, meiosis in the male cell proceeds without significant delay and produces 4 spermatozoa.

Oocyte development during the follicular growth phase *in vivo,* is characterized by a prolonged arrest at prophase I until the pre-ovulatory surge of luteinizing hormone (hereinafter designated LH). During this period of oocyte development, intracytoplasmic changes related to synthesis and storage of RNA and protein translation occurs. These processes are essential to permit oocyte meiosis, fertilization and subsequent early embryo development. It is only at the time when the follicle reaches its maximal volume that the oocyte becomes developmentally competent. The LH rise releases meiotic arrest and the oocyte resumes the first meiotic division.

Prophase I arrest in mammalian oocytes is sustained by different mechanisms at distinct times of development. Growing oocytes, enclosed in primordial up to the early antral follicles, are arrested at prophase I and are germinal vesicle breakdown (hereinafter designated GVBD) incompetent because of a functional insufficiency. Up to this point, oocytes have not synthetised the cell cycle regulatory molecules essential for meiosis progression in sufficient quantities and/or these molecules are as yet not positioned correctly within the oocyte. In contrast, prophase I arrest in GVBD competent oocytes, enclosed in antral and preovulatory follicles, is sustained by interaction with the somatic cells, which provide appropriate levels of cAMP to the oocyte via gap junctions. Alternatively, the somatic compartment transfers inhibitory factors to the oocyte. *In vivo*, gonadotropins promote oocyte maturation indirectly via effects on granulosa cells, a process mediated predominantly via the cAMP system. A rise in follicular cAMP mediates LH action to induce oocyte maturation, and intraoocyte cAMP inhibits the process. This apparent contradiction can be explained by the fact that intraoocyte cAMP level decreases subsequently through the action of phosphodiesterases resulting in the resumption of meiosis. Although it has been suggested that meiotic resumption is caused by a drop off of cAMP in the oocyte due to an interruption of the gap junctions, there is evidence that GVBD occurs prior to any detectable ionic or metabolic uncoupling between these cells. Others have shown that the levels of intracellular cAMP do not decline during meiotic resumption. Although there are still some controversies on the role of cAMP for meiosis resumption, the concept that the second messenger cAMP plays an important role in meiosis arrest in different species is widely accepted.

Supplementing culture medium with compounds that maintain elevated cAMP can prevent spontaneous maturation. This can be achieved by several compounds: cAMP analogues such as dibutyryl cAMP (hereinafter designated dbcAMP), pharmacological agents that stimulate cAMP production via adenylate cyclase (forskolin) and inhibitors of the cAMP degrading isoenzymes phosphodiesterase (hereinafter designated PDE), such as 3-isobutyl-1-methylxanthine (IBMX).

In mammalians, resumption of meiosis occurs spontaneously when GVBD competent oocytes are liberated from their follicles *in vitro.* However, in the natural cycle, LH induces oocyte maturation (GVBD) in the follicle before ovulation occurs. Furthermore, it is known that in rodents antral and preovulatory follicles, about 4 mM of a PDE inhibitor (hypoxanthine) is continuously present. In humans, by the time of ovulation *in vivo* in natural cycles, the dominant antral follicle has been growing for about 14 days and has reached a diameter of approximately 22 mm. Several studies testified that oocytes aspirated from follicles not having reached a species specific minimal diameter, are incompetent to undergo nuclear maturation.

Certain phosphodiesterases (hereinafter designated PDEs) are responsible for the breakdown of cAMP, leading to a decrease of its intracellular levels. PDE consist of a large group of proteins in which at least eleven different families have been characterized. Examples of these families are type 3 PDEs and type 4 PDEs (hereinafter designated PDE3

and PDE4, respectively). Non-selective PDE inhibitors have been used to understand the role of cAMP in the resumption of meiosis. Suppression of cAMP catabolism by using different specific PDE inhibitors demonstrated the meiosis arresting action of these chemical compounds. PDE4 is mainly involved in the metabolisation of cAMP in granulosa cells. PDE 3 has been demonstrated to act directly in the oocyte without interfering with somatic cell functions. In the rat, expression of PDE3 was specific to the oocyte. The following statement can be found in the abstract in Developmental Biology 178 (1996), 393 *et seq.*: "In isolated oocytes, spontaneous GVBD was blocked by two inhibitors of type 3 PDE". As appears from the publication, this statement applies to rats. It is stated in the abstract in J. Clin. Invest. 102 (1998), 532 *et seq.*, that "inhibitors of phosphodiesterase 3 were used to block meiosis in ovulating oocytes in rodents. By this strategy, we [i.e., the authors of this paper] demonstrated that fertilization and pregnancy could be prevented".

As appears from the above, cAMP signalling is a key factor during mammalian and amphibian oocyte maturation. In mammalians, germinal-vesicle stage (hereinafter designated GV) oocytes removed from immature antral follicles spontaneously resume meiosis *in vitro* but this process can be blocked *in vivo* and *in vitro* by certain compounds that maintain intraoocyte cAMP levels increased.

One object of the present invention is to treat human infertility.

Another object of the present invention is to improve the maturation of human oocytes.

Another object of the present invention is to improve the synchrony of nuclear, cytoplasmic and/or membranous oocyte maturation.

Another object of the present invention is to improve the fertility of oocytes.

Another object of the present invention is to improve the rate of implantation of oocytes by human *in vitro* maturation and fertilisation.

Another object of the present invention is to diminish the incidence of human preembryos with chromosome abnormalities (aneuploidy).

Another object of the present invention is to improve the cleavage rate of human preembryos.

Another object of the present invention is to improve the quality of human preembryos.

Another object of this invention is to provide a method to better synchronise cytoplasmic and nuclear maturation for IVM.

Another object of this invention is to furnish a method permitting the IVM laboratory to operate within the normal workings hours of the day.

A further object of this invention is to find a method to increases commodity of IVM practice by making the IVM independent from the time of oocyte cumulus complexes (hereinafter designated OCC) collection. (i.e., the collection time can be uncoupled from meiosis progression and subsequent fertilisation steps).

DEFINITIONS

MI is metaphase I. EGF is epidermal growth factor. GH is growth hormone. IVM is in vitro maturation. IVF is in vitro fertilisation.

DESCRIPTION OF THIS INVENTION

The compounds of general formula I below are able to inhibit PDE3. They are covered by claim 1 of European patent application having publication number 0 350 990.

PDE3s are specific enzymes present in 2 isoforms: PDE3A in myocardial, smooth muscle and in the oocyte and PDE3B functions in cells' hormonal regulation of lipolysis and glyconeogenesis (*vide*: J. Biol. Chem. 272 (1997), 6823-6826). PDE3A is the isotype of PDE3 which is present in the oocyte and regulates meiosis. The Type 3B (i.e., PDE3B) is present in other cells (fat cells) and is regulating lypolysis and neoglucogenesis.

Surprisingly, it was found that the blocking effect of a pyridazinone derivative of the general formula I

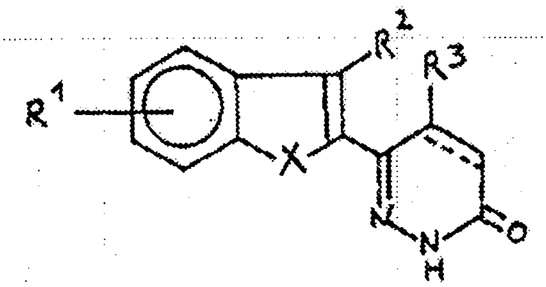

I

wherein $R^1$ represents one to four substituents, which may be the same or different and are selected from H, OH, halogen, $NO_2$ unsubstituted or C1-C4 alkyl substituted amino, C1-C4 alkyl, C1-C4 halogen substituted alkyl, O-ALK-$NR^4R^5$, C1-C4 alkoxy, whereby two substituents taken together may also represent a methylenedioxy group; $R^2$ and $R^3$ represent independently H or C1-C4 alkyl; $R^4$ and $R^5$ represent independently H or C1-C4 alkyl, or form together with the nitrogen a 5- or 6-membered ring; X represents S or O; the dotted line represents an optional bond; and their pharmaceutically acceptable salts could be reversed by washing out the compound of formula I and that a normal resumption of meiosis (normal time frame of meiosis progression and morphological aspect of the oocyte organelles) could be completed. It was demonstrated that normal offspring were born at a rate comparable to normal IVF.

For example, it has been found that favourable results are obtained by inducing an arrest in nuclear maturation concomitant with an extended *in vitro* culture period in a first culture medium (which is defined below). When cytoplasmic maturation is improved, the nuclear maturation is promoted by a second culture medium (which is defined below).

Furthermore, it has been possible to increase the normal yield of IVF/ICSI by retrieving OCC from the smaller follicles (< about 12 mm), which are normally not retrieved in a conventional treatment, but can, using this invention, now be used for IVM within a separate series of SOPs (i.e., standard operation procedures) and time schedule.

Surprisingly, when the compound of formula I is removed, meiosis will progress normally in most of the oocytes. For example, germinal vesicles (GVs) derived from follicles having a diameter from about 8 to about 12 mm will undergo normal chromatin re-modeling (surrounded nucleolus or karyosphere) during culture. Extension of the prophase arrest *in vitro* may result in cytoplasmic changes, but not in apparent nuclear changes.

By using a compound of formula I, interference with mechanisms upstream the oocyte pathway, more specifically the somatic cells cAMP-dependent pathways, can be avoided. The increased intra-oocyte cAMP results in an increase in type I isozyme protein kinase A (hereinafter designated PKA) activation, and subsequent phosphorylation of specific proteins, which are inhibitory to nuclear maturation.

As mentioned above, the present invention relates to a method for in vitro synchronisation of nuclear and cytoplasmatic maturation of GV oocytes as defined in the claims below. The method is a three step process. In the first step designated step (a), the oocytes are cultured in a first culture medium containing, *inter alia*, a compound of formula I and cytoplasmic maturation promoting substances or a co-culture system. In the second step designated step (b), a substantial part or all of the compound of formula I is removed from the oocytes. In the third step designated step (c), the oocytes are cultured in a second culture medium or a co-culture so that they become completed up to metaphase II to a desired frequency.

Preferably, the oocytes used in step (a) are collected from follicles not being too small. Usually, small oocytes from small follicles are to be cultured longer (in step (a)) than oocytes from larger follicles. Preferably, the oocytes used have a size which is at least about 70 %, preferably at least 80 %, more preferred at least about 90%, of the fully developed size of the oocytes of the species. For humans, it is preferred to use follicles having a diameter of about 4 mm to about 20 mm, preferably follicles having a diameter of about 6 mm to about 12 mm.

COCs can be retrieved from immature antral follicles in the follicular phase of the cycle before and after exposure *in vivo* to LH activity.

In a preferred embodiment of this invention, the oocytes used in step (a) are collected from follicles which were aspirated from the animal/human (cycling animal/human or not) before any hormone treatment that could effect either folliculogenesis and/or oogenesis). In another preferred embodiment of this invention, the hormone treatment of the woman from which the oocytes originates were performed with a steroid, a gonadotrophin, a GnRHanalogue, clomiphene citrate, tamoxiphen, an insulin sensitiser (e g. metformin), an aromatase inhibitor or any of the preceding drugs alone or combined. In another preferred embodiment of this invention, the oocytes are collected from follicles which were aspirated from the animal after any hormone or drug treatment that effects upon folliculogenesis and/or oogenesis. In another preferred embodiment of this invention, the hormone or drug treatment is performed with a gonadotropin, a GnRHanalogue, clompihene citrate, tamoxiphen, LH, HCG, a steroid or a steroid-like substance, an LHRH analogue, an aromatase inhibitor, an insulin sensitizing agent, and any analogue or combination thereof. In another preferred embodiment of this invention, the hormone used is hCG, LH or any compound which might induce similar effects.

The medium used for culturing the oocytes is any medium containing the necessary nutrition components and which does not contain a substantial amount of compounds having an adverse influence of the cultivation of said oocytes. Such media are known to the skilled art worker. Furthermore, the skilled art worker is familiar with convenient culturing conditions such as temperature, osmolarity, humidity, time and the like.

Conveniently, the first medium contains as basis a bicarbonate buffer system (allowing a pH value of about 7.4 by incubation in a $CO_2$ incubator) and which has an osmolarity of about 285 mOsm per kilogram water. Culture is done at the adequate temperature for the species (about 37°C for the human) and at a humidity of about 10%. Use can be made of several commercially available defined culture media or tissue culture media where a human serum albumin (which optionally is prepared by genetic engineering) is added as protein source. Examples of adequate media to use are DMEM or TC-199 supplemented *inter alia* by insulin (e.g., 1 ng/ml), transferrin (e.g., 5 microgr/ml), selenium (e.g., 5 ng/ml), 0.1% HSA, pyruvate, e.g., 23 microMolar, glutamine, e.g., 2mM, and/or 17β-estradiol (hereinafter designated

beta-E2).

In a preferred embodiment, the content of the compound of formula I in the first medium is in the range from about 0.1 to about 100 micromolar which, of course, can vary depending upon the potency of the specific compound of formula I used.

Conveniently, one or more cytoplasmatic promoting factors are added to the first medium. Specific examples of cytoplasmatic promoting factors are EGF *(*vide Fertil. Steril55 (1991), 1000-1004; and Zygote 5 (1995), 345), GH *(*vide Mol. Reprod. Dev. 45 (1996), 372-377), gonadotropin combinations *(*vide Fertil. Steril. 52 (1989), 319-324; and Mol. Reprod. Dev. 45 (1996), 218-224), promoters of glutathione synthesis (vide Theriogenology 53 (2000), 761-771), meiosis activating sterols (vide Biol. Reprod. 58 (1998), 1297-1302), activin and inhibin (each alone or in combination) *(*vide Biol. Reprod. 58 (1998), 558-565; and Biol. Reprod. 56 (1997), 1559-1564), the analogues of the foregoing alone or in any combinations of the aforementioned compounds.

In step (a), the oocytes are, conveniently, incubated in a culture vessel which, conveniently, is covered by a minimal volume of oil compared to the volume of medium.

In step (a), the oocytes are to be culturing for a time period sufficient for cytoplasmatic maturation to progress or to allow for normal working hours during the IVF procedure.

One way of judging whether a time period is sufficient is to evaluate the outcome of the subsequent oocyte fertilization and embryo culture steps. The outcome of the subsequent oocyte fertilization step can be judged by any skilled art worker, e.g., number of 2 cells, the synchrony of nuclear, cytoplasmic and/or membranous oocyte maturation, the fertility of oocytes, the rate of implantation of oocytes by human *in vitro* maturation and fertilisation, the incidence of human preembryos with chromosome abnormalities (aneuploidy), the cleavage rate of human preembryos, and the quality of human preembryos. Outcome of fertilisation is usually checked by percent of 2 cells/inseminated oocyte. Outcome of embryo culture is judged by morphology of blastomeres (regularity or fragmentation), cleavage time intervals, blastocyst morphology and capacity to hatch.

Another way of judging whether a time period is sufficient for the cytoplasmatic maturation to progress is to use a validated molecular biology method (custom microarray technique) to access the absence or presence of selected markers expressed by the cumulus cell.

An example of a specific compound of formula I is 4,5-dihydro-6-(5,6-dimethoxybenzo[b]thien-2-yl)-5-methyl-3(2H)-pyridazinone or 4,5-dihydro-6-(5,6-dimethoxybenzo[b]-thiophene-2-yl)-5-methyl-3(2H)-pyridazinone (hereinafter designated DDMP (alternatively designated ORG 9935)) and having the formula:

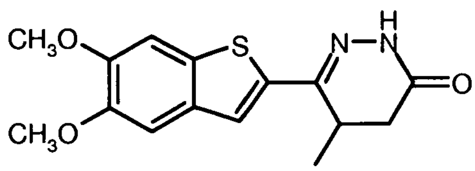

The concentration of the DDMP in the first medium is conveniently in the range from about 1 μM to about 100 μM.

Examples of domestic animals are dogs, cats, cows, pigs, horses, and sheep. Examples of primates are monkeys and humans, preferably humans.

In a preferred embodiment of this invention, the oocytes used for culturing in the first medium are oocytes which have been stored in an oocyte collection medium being a $CO_2$-independent medium containing compound of formula I.

In a preferred embodiment of this invention, the oocyte has been placed in said oocyte collection medium not later than 4 hours, preferably not later than 2 hours, even more preferred not later than 1 hour, and even more preferred not later than ½ an hour, after said oocyte has been removed from the woman.

In a preferred embodiment of this invention, the culturing according to step (a) takes place for a time period sufficient for cytoplasmatic maturation to be improved. In another preferred embodiment of this invention, the culturing according to step (a) takes place for a time period sufficient for cytoplasmatic maturation to be completed.

In a preferred embodiment of this invention, the oocytes cultured in step (a) are GV oocytes.

In a preferred embodiment of this invention, the first culture medium contains EGF, GH, gonadotropin combinations, promoters of glutathione synthesis, meiosis activating compounds, combinations of activin and inhibin, the analogues of the foregoing or combinations of the aforementioned compounds.

In a preferred embodiment of this invention, the compound of formula I is DDMP.

In a preferred embodiment of this invention, the culturing time in step (a) is at least about 4 hours, preferably at least about 8 hours, more preferred at least about 12 hours, even more preferred at least about 24 hours, more preferred at least about 48 hours and even more preferred at least about 72 hours.

In a preferred embodiment of this invention, the first culture medium is minimally covered by a thin layer of an oil so that the ratio between the oily phase and the aqueous phase is less that about 2:10 (vol/vol), preferably less that

about 1:10 (vol/vol). In another preferred embodiment of this invention, the oocyte collection medium is covered by oil.

In a preferred embodiment of this invention, there are cumulus cells around the oocyte used in step (a).

Conveniently, **the second medium** contains the same basis components as the first medium (see above) to which is added HCG, LH or MAS, analogues of these compounds alone or in any combination.

In a preferred embodiment of this invention, the oocytes of step (a) are washed in order to remove the compound of formula I or decrease the amount thereof, so that any compound of formula I present has no substantial effect on the arresting of the oocytes.

In a preferred embodiment of this invention, the washing of the oocytes from step (a) is performed so that the concentration of the compound of formula I after the washing out of the compound of formula I in the second medium is less than about 10%, preferably less than about 5 %, more preferred less than about 1% of the concentration of the compound of formula I in the first medium.

In the **third step,** the oocytes cultured in the second medium can be covered by an amount of oil.

In a preferred embodiment of this invention, there are no or substantially no cumulus cells around the oocyte in step (c).

In a preferred embodiment of this invention, the nuclear maturation is stimulated by adding HCG and/or EGF and/or a MAS compound to the second culture medium in step (c). In case there are no cumulus cells present around the oocyte (most times after a long culture period cumulus drops off the oocyte), HCG or EGF cannot effect on oocyte. However, MAS compounds can in such cases.

In a preferred embodiment of this invention, the desired frequency in step (c) for nuclear maturation to be completed up to metaphase II is at least about 30 %, more preferred at least about 50 %, even more preferred at least about 70 %, and preferably at least about 90 %.

In a preferred embodiment of this invention, after step (c), the oocytes are fertilized with sperm, preferably using ICSI.

The present invention makes it possible to steer away from working in the weekends or in the evening or night.

The nuclear maturation has been completed when there is a first polar body extrusion. This can, for example, be determined microscopically.

Metaphase II is the stage where the first polar body is present in perivitteline space.

In a preferred embodiment of this invention, the oocytes used in step (a) have been stored in an oocyte collection medium which, conveniently, is a $CO_2$-independent medium containing a compound of formula I. For example, it could be a HEPES buffered medium or a PhoSphate medium (for example Leibovits medium). The oocytes are stored in this oocyte collection medium as soon as they come out of the follicle. The reason for this is that, otherwise, the oocytes might be collected in distant places (satellite collection centres and field conditions and are then transported to the IVF/ICSI laboratories). In the latter case, if time goes over, there might not be a sufficient inhibition of oocyte maturation present. The use of such an oocyte collection medium is to avoid that oocytes start to resume meiosis, especially if they are of the more denuded-type from cumulus cells.

The mentioning herein of references is no admission that they constitute prior art.

Herein, the word "comprise" is to be interpreted broadly meaning "include", "contain" or "comprehend" (*vide*, for example, EPO guidelines C 4.13).

This invention is further illustrated in the following experiments which, however, are not to be construed as limiting the scope of protection.

The features disclosed in the foregoing description and in the following may, both separately and in any combination thereof, be material for realizing this invention in diverse forms thereof.

Example 1

MATERIAL AND METHODS

Collection of immature oocytes

Patients included in this study were undergoing fertility treatment and were primed with variable doses of gonadotropins while being carefully monitored using serum hormone and vaginal ultrasound monitoring (Human Reproduction 7 (1992), 49 *et seq.*). Collection of oocytes cumulus complexes (COCs) from different follicle sizes was performed as follows: 1 °) during laparoscopic surgery, 2°) aspirated from patients with stimulated ovaries undergoing fdlicle reduction before intrauterine insemination (to avoid multiple gestation due to multiple ovulation) (Albano et al, 2001) or 3°) during ovum pick up after having aspirated the larger follicles (> 15 mm) destinated for IVF treatment (Human Reproduction 7 (1992), 49 *et seq.*). Basically, these aspirations yielded two types of COC: a first group that had never been exposed to LH activity (pre-hCG) and a second group of follicles 36 h after an exposure to hCG (injected) or endogenous LH (spontaneous LH peak). When possible, follicle diameters were recorded by vaginal ultrasound measurement. COCs

used in this study were retrieved from follicles with diameters between 6 and 14 mm.
Human oocytes aspiration was performed by ultrasound-guided transvaginal follicle aspiration using a single lumen needle (Cook, K-OPS-1230-VUB, Switzerland AG) after paracervical infiltration with a local anesthetic. The aspiration pressure used was reduced from the usual 100 mmHg for recovery of mature oocytes to 60 mmHg. Follicles were flushed using HEPES-buffered Earle's medium at least three times.

*Classification of Cumulus oocyte complexes (COCs)*

The COCs were evaluated immediately after aspiration and after 24 hours and 48 hours culture and classified as follows: type (I), compact mass of 3-5 layers of granulosa cells; type (II), expanded distal layers of granulosa cells (cumulus), but a compact proximal granulosa cell layer; type (III), expanded distal (cumulus) and proximal (corona cells) layers of granulosa cells; and type (IV) partially denuded oocytes due to expanded granulosa cells.

Culture of COCs

At the time of oocyte retrieval only the COCs classified as I or II were placed in culture.
Twenty-four hours later, oocyte stages were recorded and oocytes showing a clear polar body extrusion were removed from culture. After 48 hours, all oocytes were assessed and GV, GVBD and polar body extruded ones were recorded.
The basal medium used for oocyte culture was D-MEM (D-5280, Life Technologies, Merelbeke, Belgium) with 2 mM Glutamax-I, $NaHCO_3$ (2g/L) supplemented with 10 mIU/ml FSH (Gonal-F®, kindly donated by Ares Serono, Geneva, Switzerland), 100 ng/ml IGF-I (R&D, UK), 5 ng/ml insulin, 5 ng/ml selenium, 5 μg/ml transferrin (Life technologies, Merelbeke, Belgium) and 0.1% human serum albumin (CAF-DCF, Brussels, Belgium). 17β-$E_2$ (Sigma, Bomem, Belgium) was diluted in ethanol and added to oil to reach a final concentration of 1μg/ml in the 10 μl medium droplets underneath (this concentration was measured using specific radioimmunoassay). Microdroplets of 10μl medium, were covered with 2ml of oil. A refreshment of 5μl conditioned medium was performed each 24 hours. All cultures were carried out at 37°C in humidified atmosphere in an incubator gassed with 5% $CO_2$ in air.
Schematic diagram of the study is shown in Fig. 1.

*Oocyte inhibition and reversal of meiosis*

DDMP was added at a 10 μM final concentration (0.1 % DMSO). Directly after aspiration, single COCs were washed in flushing medium (D-MEM with HEPES) and placed into 10 μl droplets under oil. COCs were randomised over two culture conditions: with and without DDMP. Oocytes cultured with DDMP for 24 hours, 48 hours and 72 hours were studied for reversal of meiosis arrest by washing out the inhibitor. Reversal of PDE 3 inhibition was performed by mechanical denudation of the remaining somatic cells and placing the oocytes in the conditioned medium for an additional 24-30 h.

*Oocyte preparation for electron microscopy (hereinafter designated EM)*

During evaluation at 24 hours and 48 hours culture, part of the oocytes with and without DDMP were fixed for EM analysis.
A total of 48 oocytes (4 at time of retrieval, 21 after 24 hours, 20 after 48 hours and 3 after 72 hours culture) were fixed in glutaraldehyde 2.5% in cacodylate buffer at pH 7.3 for at least 2 hours at 4°C at time 0 h, 24 hours and 48 hours of maturation. All oocytes were embedded individually. Afterwards, they were postfixed in $OsO_4$ 1% in $H_20$ for 30 min, stained with uranyl acetate 2% in veronal buffer for 60 min, dehydrated through graded alcohols, embedded in SPURR (Taab; Bodson, Liège, Belgium) and left to polymerize overnight at 70°C.
Semithin sections of 0.5 μm and ultrathin sections of 75 nm were made at approximately 30 levels (range 15-35). The semithin sections were mounted on glass slides and stained with toluidine blue for light microscopical guidance. The ultrathin sections were transferred on wide single slot copper grids (Agar Scientific LTD, Stansted, UK), coated with a polyvinyl formal film (Formvar, Laborimpex, Brussels, Belgium), stained with lead citrate and examined using a Zeiss transmission electron microscope type EM 9S2.

Oocyte preparation for Confocal Laser Microscopy

For localization of microfilaments, 39 polar body extruded oocytes (25 oocytes 48 hours after maturation in control medium and 14 oocytes after inhibitor removal) were fixed with 2.5% glutaraldehyde in cacodylate buffer for at least 2 hours, washed with PBS/1% BSA, placed into 0.1% Triton X-100 for 5 min and rinsed again with PBS. Afterwards,

oocytes were placed into Texas red-labeled phalloidin staining f-actin diluted 1:40 PBS for f-actin and Pico green 1:2000 PBS for DNA staining.

*Statistical analysis*

Evaluation of cumulus morphology expansion in culture related to follicle treatment, resumption of maturation, kinetics of GVBD and polar body extrusion were analysed using the $\chi^2$ test and Fisher's exact test for comparison between control and DDMP treated groups. Statistical significance was considered when $P< 0.05$.

RESULTS

Oocyte recovery rates in the different groups

A total of 139 follicles were aspirated from 30 patients before any hCG administration. The follicles between 6 and 12 mm (45.3%) yielded an oocyte recovery rate of 80.9 % (mean ±SD of oocytes per patient: 3.2 ±2.2; range 1-9). The aspirated follicles larger than 12 mm (54.7%) yielded an oocyte recovery rate of 35.5 % (mean ±SD of oocytes per patient: 1.7 ±0.9; range 1-4).

A total of 128 follicles between 6 and 12 mm were aspirated from 27 patients at 36 hours after hCG administration. These yielded an oocyte recovery of 50.0% (mean ±SD of oocyte per patient: 2.4 ±1.5; range 1-6). A total of 54 follicles between 13 and 15 mm were aspirated from 9 patients with an endogenous LH peak a day before follicle aspiration. These yielded an oocyte recovery of 37.0% (mean ±SD of oocyte per patient: 2.2 ±0.8; range 2-4).

MORPHOLOGICAL CHARACTERISTICS OF COCS

CUMULUS CELL EXPANSION AT THE MOMENT OF RETRIEVAL AND DURING CULTURE IN CONTROLS (FIG. 2)

*COC retrieved pre-hCG administration*

At retrieval there was a high proportion of COCs with compact cumulus morphology (type 1) (83.8%). After 24 hours, in controls the proportion of types I COCs decreased to 10.8%. At this time, 51.4% and 29.7% COCs were classified as type II and III, respectively. At 48 hours culture, 40% of oocytes had lost their connections with the cumulus cells (type IV).

*COC retrieved post-hCG administration*

This group comprises the patients in whom hCG was administered for 36 hours (normal IVF/ICSI procedure) and those patients who had an endogenous LH surge.

The COCs retrieved from the post-hCG patients presented a significantly lower proportion of type I COCs compared to pre-hCG patients (45.0%) ($p< 0.01$). Twenty-four hours later, almost all COCs presented an expanded cumulus (42.5% of type II and 35.0% type III) and 20.0% had already lost the connections cells-oocyte. Finally at 48 hours culture, a significantly higher proportion of oocytes had lost their connections with the cells compared to the cultured ones from the pre-hCG group (66.7%) ($p<0.05$).

CUMULUS CELL EXPANSION IN THE PRESENCE OF THE DDMP (FIG. 3)

*COC retrieved pre-hCG administration*

From the retrieved COC from pre-hCG patients, 74.4% were classified type 1. After 24 hours, the proportion of type I decreased to 25.6%. At this time, 56.4% and 15.4% COCs were classified type II and III, respectively. Finally at 48 hours culture, 22.2% of oocytes had lost their connections with the cumulus cells.

*COC retrieved post-hCG administration*

The proportion of type I COCs retrieved from the post-hCG patients was significantly lower (48.8%) compared to those retrieved from pre-hCG patients ($p<0.05$). Twenty-four hours later, only 4.7% from post-hCG cumuluses remained type I. This constitutes a significantly lower proportion than the pre-hCG cumulus ($p<0.05$). There were 57.1 % of type II and 30.9% type III cumuluses (not significantly different from pre-hCG). After 24 hours, a similar proportion of oocytes as in the pre-hCG group had lost connection with cumulus cells (7.1 %). Finally at 48 hours culture, 62.1% of oocytes had lost connections with the somatic cells, this is higher than COCs retrieved from the pre-hCG group ($p<0.05$).

The expansion and mucification pattern of COC before and during culture was not modified by the presence of PDE 3 in medium.

PRESENCE OF DDMP IN CULTURE: TIMED ANALYSIS OF NUCLEAR MATURATION *EVALUATION AT 24 HOURS OF CULTURE*

*COC retrieved pre-hCG administration (Fig. 4)*

A total of 39 COCs at GV-stage were cultured in presence of DDMP and 37 COC without (= control). After 24 hours culture, all oocytes remained at GV-stage when DDMP was present in the medium in contrast to only 24.3% in control medium (p<0.001). In controls, four oocytes (10.8%) had a polar body extruded. A large proportion of oocytes (64.9%) did not show clear presence of a polar body into the perivitelline space (not shown in Fig.).

*COCs retrieved post-hCG administration (Fig. 5)*

A total of 42 immature COCs were cultured in the presence of DDMP and 40 COCs in control. COCs retrieved from exogenous (hCG) and endogenous (spontaneous) LH peaks were grouped in the same tables and Figures. After 24 hours culture, 92.9% of oocytes remained at GV-stage in DDMP medium while only 22.5% in controls (p<0.001). Of those oocytes, which did not arrest at GV-stage despite DDMP, only 2.4% extruded a visible polar body (PB) (not shown in graph). In controls, 10.0% had extruded a polar body (PB). The proportion of oocytes in which it was impossible to visualize the nucleus to be sure of the presence of PB was 67.5 % in controls.

EVALUATION AT 48 HOURS OF CULTURE

*COCs retrieved pre-hCG administration (Fig. 4)*

COCs in which the nuclear stage was not clearly visualized were mechanically denuded. Those that were fixed for EM analysis had their maturational stage determined afterwards (on the semithin sections). Oocytes cultured in presence of the DDMP, remained for 88.9% at the GV-stage in contrast to only 19.3% in controls (p<0.001). In the presence of DDMP, only 5.5% of oocytes were GVBD, 2.8% were PB and in 2.8% the stage could not be determined. In controls, 12.9% were GVBD, significantly more oocytes had a PB extruded (64.5%) (p<0.001) and 3.2% had a non determinable stage. The relation between follicle diameter recorded and number of polar body extruded oocytes after culture is shown in Table 2 **Table 2.** Number of oocytes reaching normal polar body extrusion after 24 or 48 hours of in vitro culture in control medium. Data grouped according to follicle size at retrieval and treatment by hCG or not

| Follicle size at retrieval | Patient treatment | Number of oocytes at time of retrieval | Number of PB extruded oocytes between 24-48 hours culture; (% of total) | |
|---|---|---|---|---|
| > 12 mm | Pre-hCG | 13 | 11 | 84.6 |
| | Post-hCG | 11 | 11 | 100.0 |
| ≤ 12 mm | Pre-hCG | 24 | 13 | 54.1 |
| | Post-hCG | 29 | 21 | 72.4 |

*COCs retrieved post-hCG administration (Fig. 5)*

COCs cultured in presence of DDMP showed a significant greater proportion of oocytes at the GV-stage (83.3%) compared to controls (6.0%) (p<0.001). In DDMP, 8.3% of oocytes were GVBD, only 5.6% were PB and 2.8% had a not determined stage. In controls, 12.1% were GVBD and a significant greater proportion of oocytes had a PB extruded (81.8%) (p<0.001). The relation between polar body extruded oocytes and follicle diameter is shown in Tale 2. It was also observed (from the relation cumulus typing-nuclear progression) that from the COCs classified as type II at the time of retrieval from post-hCG patients (51.2%), 77.6% of them could remain arrested at the GV-stage for 48 hours culture. This shows that the inhibitor was effective even on the oocytes with a more expanded cumulus morphology at the time of oocyte retrieval.

EVALUATION AT 72 HOURS CULTURE

After 72 hours in the presence of DDMP, 16 out of 19 (84.2%) oocytes from pre-hCG and 10 out of 15 (66.6%)

from post-hCG patients were still arrested at the GV-stage.

*Reversibility after arrest for 48 or 72 hours.*

After 48 hours culture in DDMP, a total of 22 GV-stage oocytes were mechanically denuded from cumulus cells. Inhibitor was washed out (11 from pre-hCG and 11 from post-hCG group) and oocytes were further cultured for 24-30 h in control medium. From the pre-hCG patients group, 4 progressed to GVBD but 1 arrested at this stage and 3 of them extruded the polar body at 24-30h. From the post-hCG patients group, 11 oocytes progressed to GVBD but 5 arrested at this stage and 6 extruded the polar body (Table 3).

**Table 3.** Number of oocytes at different stages of meiotic progression (GV, GVBD, PB) as observed at 24-30 hours after removal of DDMP from the maturation medium. Data grouped by duration of culture days in DDMP supplemented medium and by type of patient treatment (hCG or not)

| Day of Inhibitor removal | Patient treatment | Number of oocytes | Oocyte stage 24-30 hours after DDMP removal | | |
|---|---|---|---|---|---|
| | | | GV | GVBD | PB (%) |
| 2 | pre-hCG | 11 | 7 | 1 | 3 (27) [a] |
| | post-hCG | 11 | - | 5 | 6 (54) [c] |
| 3 | pre-hCG | 13 | 6 | 1 | 6 (46)[a] |
| | post-hCG | 10 | - | - | 10 (100) [bd] |

[a] and [b] denotes a significant difference by *Fisher*'s exact *test* ($p < 0.01$).
[c] and [d] denotes a significant difference by $\chi 2$ *test* ($p < 0.05$).

After 72 hours in the presence of DDMP, 13 oocytes from pre-hCG and 10 from post-hCG patients were denuded and inhibitor was washed out. After 24 hours to 30 hours culture in conditioned medium, from the pre-hCG patients group, 7 progressed to GVBD but 1 arrested at this stage and 5 extruded a PB. From the post-hCG patient group, 10 oocytes achieved PB extrusion 24-30h after inhibitor removal (Table 3) which was significantly higher compared to the pre-hCG group (p<0.05). The proportion of oocytes with PB extrusion after inhibitor removal from the post-hCG group was also significantly higher when oocytes were cultured for 72 hours in DDMP culture compared to 48 hours (p<0.05).

*Light and electron microscopy*

In order to analyse the effect of PDE 3 inhibition on nuclear and cytoplasm organization, ultrastructural observation was performed at the different culture times in oocytes arrested or not by DDMP. The oocytes analyzed did originate from follicles with diameter between 8-12 mm. Numbers and conditions of oocytes analysed by EM are listed in Table 1.

**Table 1.** Number of oocytes analysed by EM in the different maturation stages (GV, GVBD, PB) at time of retrieval and 24, 48 and 72 hours after culture. Oocytes analyzed are grouped by type of patient treatment and culture conditions

| | | | Number of oocytes analyzed at different times of culture in the different stages of nuclear maturity | | | | | | |
|---|---|---|---|---|---|---|---|---|---|
| Patient treatment | Number of oocytes at time of retrieval | Culture conditions | After 24 hours culture | | | After 48 hours culture | | | After 72 hours culture |
| | | | GV | GVBD | PB | GV | GVBD | PB | GV |
| Pre-hCG | 2 | Control | | 2 | 4 | | | 9 | |
| | | Inhibitor | 3 | | | 4 | | 1 | 3 |
| Post-hCG | 2 | Control | 2 | 1 | 4 | | 1 | 2 | |
| | | Inhibitor | 3 | 1 | 1 | 3 | | | |
| Total | 4 | | 8 | 4 | 9 | 7 | 1 | 12 | 3 |

Oocyte morphology at time of retrieval - 0 hour

Semithin and ultrathin sections revealed that the GV of these oocytes was located centrally (three oocytes) or close to the plasmalemma (in one oocyte). The nucleus consisted of an envelope formed by two layers of membrane, containing one dense compact nucleolus associated with inter-chromatin granular complexes and extranucleolar chromatoid bodies on the periphery of nucleoli (Sathananthan, 1985). The nucleoli are regularly shaped, round or oval, homogeneous and formed by fibrillar granules. In all oocytes analysed the formation of nucleolus-associated chromatin was observed as a continuous mass (karyosphere), which normally exists in oocytes obtained from antral follicles of 8 mm and larger (Tesarik et al, 1983). The karyosphere, also called as surrounded nucleolus (SN), occupied an excentric position in the nucleus and consists of loosely packed fibrills in contact to the nucleolus (Fig. 6b). The presence of karyosphere was independent of the position of the nucleus in the oocyte (only in one oocyte the nucleus was at the periphery).

At the level of organization of the cytoplasm, a characteristic of all the oocytes analyzed at retrieval was the presence of areas devoid of organelles at the cortex and intermediate cytoplasm. Clumps of mitochondriae were found distributed throughout the cytoplasm (Fig. 6a,c). At the surrounding of the GV aggregates of mitochondria were intermingled with vesicles of different diameter. Cortical granules were found dispersed throughout the cytoplasm and few were observed under the oolemma of the oocyte forming a single layer.

Microvilli were noticed extending from the plasma membrane through the inner cortex of the zona pellucida (ZP). Numerous cell projections penetrated the zona pellucida to the oolemma. The granulosa cells (GC) appeared as oval or round cells apposed to the ZP with numerous cellular projections crossing the ZP reaching the oolemma. Cells displayed a compact arrangement and tightly adhered to each other by closely apposed plasma membranes resembling gap junctions (Fig. 6d). The nuclei of the cells are excentrically located. Granulosa cells of the COC did not show signs of pyknosis.

*Morphology of COCs after 24 hours culture without inhibitor*

Electron microscopy analyses of oocytes cultured in control medium demonstrated that *in vitro* maturation was associated with reorganization of mitochondria and smooth endoplasmic reticulum (SER) which appear as round vesicles into cytoplasm. This reorganization was not different for oocytes retrieved pre or post-hCG stimulation. Mitochondriae are spherical or oval and are dispersed throughout the cytoplasm or around aggregates of SER. These aggregates form characteristic complexes with peripheral mitochondria in the maturing human oocytes (vide Ann. NY Acad Sci. 442 (1986), 251 *et seq.*. and Prog. Clin. Biol. Res. 296 (1989), 273 *et seq.*) and were predominantly present in the PB-extruded ones.

Cortical granules (CG) showed a discontinuous distribution on the surface of the GVBD oocytes and appeared in one to two layers at the cortex of PB-extruded oocytes.

The oocyte that remained at the GV-stage after 24 hours in control medium had the same type of nucleus (with presence of karysphere) and organelle distribution as for the GVs fixed and analyzed at time of retrieval (Fig. 7b).

After 24 hours of culture cumulus cells were more elongated, retracted from the zona pellucida and partially detached from the oocyte. The nuclei of the cells were displaced to the periphery of the cell at the opposite side of the elongation (Fig. 7a, c).

*Morphology of COC after 24 hours culture with inhibitor*

The GV of the oocytes were centrally located with presence of a karyosphere around the nucleoli of all oocytes. The nuclear membranes of all oocytes had remained intact (Fig. 8b).

All oocytes showed signs of immature cytoplasm similar to the ones fixed at time of retrieval. Mostly, the mitochondriae were found distributed in clumps throughout the cytoplasm (Fig. 8c). In the surroundings of the GV, aggregates of mitochondria were intermingled with vesicles of different diameter. Few cortical granules were found dispersed throughout the cytoplasm and beneath the oolemma of the oocyte, forming a single layer. A single GV-stage oocyte (retrieved from a 9mm post-hCG follicle aspiration) had no presence of karyosphere in the nucleus and a more homogeneous distribution of organelles in the cytoplasm. The cytoplasm of the GVBD oocyte analysed showed an inhibition of the distribution of organelles, which were aggregated as in the cytoplasm of a GV-stage oocyte. The oocyte that underwent PB extrusion had the same cytoplasmic morphology as the control ones.

The cumulus cells started to expand and loose contact with the oocyte. Cumulus cells started their elongation, although some cells were still round or oval. The nuclei of these cells did not yet migrate to the periphery (Fig. 8a).

*Morphology of COC after 48 hours culture without inhibitor*

The PB-extruded oocytes analysed after 48 hours culture in controls formed the meiotic spindle perpendicular to the oolemma (Fig. 9a). The organelles, mitochondriae and SER were spread throughout the ooplasm as vesicles or as aggregates of tubular elements. The CG formed one to three layers under the oolema, and in one matured oocyte (pre-hCG), numerous CG were conglomerated beneath the oolema at the place of the meiotic spindle. Some swollen SER vesicles were observed perhaps as a sign of oocyte ageing (Sathananthan, 1982) (Fig. 9b). In all oocytes few CG could also be observed throughout the cytoplasm. An increase of vesiculation was not observed in any of these oocytes.
Most oocytes had their surrounding cells more dispersed or completely detached from the zona and less transzonal processes appeared from cumulus cells into the ZP.

*Morphology of COC after 48 hours and 72 hours culture with inhibitor*

The GV-stage oocytes fixed and analyzed after 48 hours and 72 hours culture in the presence of the inhibitor showed a centrally located nucleus with presence of karyosphere around the nucleoli in almost all oocytes (Fig. 10a). One GV-stage oocyte at 48 hours (retrieved from one 8 mm pré-hCG follicle aspiration) had no presence of karyosphere in the nucleus and a more homogeneous distribution of organelles in the cytoplasm. The nuclear membranes of all oocytes had remained intact. The organelles were still aggregated, although in some oocytes the mitochondria started to dissociate from their aggregates. Few cortical granules were found dispersed throughout the cytoplasm and under the oolemma of the oocyte forming a single layer (Fig. 10b). The cumulus cells were more dispersed or completely detached from zona with less transzonal processes through the ZP. The oocytes that underwent maturation (GVBD and PB) had the same cytoplasmic morphology as the controls.
After 72 hours, the oocytes showed signs of vacuolization in the ooplasm, resulting from swelling of SER (Fig. 11 a, b). Microvilli were less evident and zona was more homogeneous with no transzonal processes from cumulus cells, indicating a complete loss of connections with the oocyte (Fig. 11 b).

*Confocal Laser Microscopy for microfilaments and chromosome analysis*

A total of twenty oocytes (7 out of 8 pré-hCG; and 13 out of 17 post-hCG) which were stained after 48 hours culture in control medium presented a metaphase II (MII) plate of which 80.0% had well-aligned chromosomes and positioned perpendicular to the oolema. One oocyte presented chromosomes dislocated from the M II plate (Fig. 12). Four oocytes had nuclei forming a clump of chromosomes at the place of metaphase plate, probably as a sign of ageing. Microfilaments were observed in the scanned levels of oocytes with a more prominent staining at the optical sections close to the site of first polar body extrusion described as generalized pattern (Terada et al, 1995). No superposed microfilaments of actin were observed at the site of polar body extrusion, showing a homogeneous staining at this level.
Twelve oocytes presented the M II plate 24-30 hours after inhibitor removal (3 out of 3 pre-hCG, and 9 out of 11 post-hCG) in which 83.3% had were well-aligned chromosomes and positioned perpendicular to the oolema (Fig. 13). Two oocytes had nuclei forming a clump of chromosomes at place of metaphase plate. The generalized aspect of microfilaments was also observed in those oocytes with homogeneous prominent staining at the level of the polar body extrusion site (Fig. 13).

CONCLUSIONS FROM THE ABOVE DATA

Use of DDMP allowed demonstrating that PDE3 significantly participates in the regulation of human oocyte maturation. Used at a dose of 10 μM DDMP consistently blocked resumption of meiosis *in vitro* in COCs extracted from antral follicles for at least 48 hours culture without ultrastructural morphological signs of oocyte degeneration. Nuclear arrest could be held on up to 72 hours of culture. After inhibitor removal, the arrested oocytes were capable of resuming meiosis within the normal time frame.
We experienced that, by using a selective DDMP, meiotic arrest in GV could be maintained effectively for a period of at least 48 hours. It was also observed that, in this serum-free culture medium, cumulus cells start to loose connections to the oocyte after 24 hours. In oocytes collected after hCG pre-treatment, breakdown of connections is even more rapid and transzonal processes can be kept only for a maximal period of about 48 hours. The DDMP itself had no influence on this progressive loss of coupling between somatic cells and oocyte. This emphasises the advantage of using a selective inhibitor acting directly into the oocyte cAMP metabolism via the intra oocyte PDE3A bypassing the needs for somatic cell-dependent cAMP generation to arrest the nuclear maturation.
Using a selective DDMP to block cAMP degradation in the oocyte produced normal morphological signs of nuclear stagnation of GV oocytes for 72 hours. The nuclear membrane remained intact and unfolded, the surrounding nucleus chromatin configuration (SN or karyosphere) was persistent during arrest and the germinal vesicle was most

often located in a central position. It has been reported that karyosphere formation reflects the state of oocyte nucleus being prepared for ovulation with extinction of transcriptional processes. The karyosphere was detectable in the nucleus of the oocytes when they were still surrounded by a compact cumulus cell mass. Several transzonal processes onto the oocytes were observed with a heterogeneous zona and microvilli were still present and abundant.

Prolonged nuclear arrest by DDMP in our *in vitro* maturation medium seemed to affect the migration of organelles in the GV-stage oocytes. The organelles start to dissociate from the aggregates after 48 hours in DDMP culture, even in GV-intact oocytes. In the DDMP arrested oocytes, cortical granules had formed a characteristic single layer, while in contrast controls had their cortical granules still conglomerated.

Safety studies regarding the use of DDMP on oocytes were done in rodents; it was confirmed that blocking effects are fully reversible in isolated oocytes. After pharmacological arrest of human oocytes for 48 or 72 hours, the meiotic cycle can be reinitiated by washing out the DDMP. Depending on follicle pretreatment conditions (HMG stimulation alone or in combination with an HCG stimulation), 46% to 100% of oocytes could normally progress through meiosis with formation of a metaphase II plate with fidelity of chromosome segregation.

Example 2

Herein, the term MAS compound designates compounds which mediate the meiosis of oocytes. More specifically, a MAS compound is a compound which in the test described below in this example has a percentage germinal vesicle breakdown (hereinafter designated GVB) which is significantly higher than the control. Preferred MAS compounds are such having a percentage GVB of at least 50%, preferably at least 80%.

Examples of MAS compounds are mentioned in WO 96/00235, 96/27658, 97/00884, 98/28323, 98/54965 and 98/55498, more specifically in Claim 1 thereof.

The evaluation of whether a specific compound is a MAS compound or not can, for example, be made using the following test:

Oocytes were obtained from immature female mice (C57BL/6J x DBA/2J F1, Bomholtgaard, Denmark) weighing 13-16 grams, that were kept under controlled temperature (20-22 °C), light (lights on 06.00-18.00) and relative humidity (50-70%). The mice received an intraperitoneal injection of 0.2 ml gonadotropins (Gonal-F, Serono) containing 20 IU FSH and 48 hours later the animals were killed by cervical dislocation. The ovaries were dissected out and the oocytes were isolated in Hx-medium (see below) under a stereomicroscope by manual rupture of the follicles using a pair of 27 gauge needles. Spherical oocytes displaying an intact germinal vesicle (hereinafter designated GV) were divided in cumulus enclosed oocytes (hereinafter designated CEO) and naked oocytes (hereinafter designated NO) and placed in $\alpha$-minimum essential medium ($\alpha$-MEM without ribonucleosides, Gibco BRL, Cat. No. 22561) supplemented with 3 mg/ml bovine serum albumin (BSA, Sigma Cat. No. A-7030), 5 mg/ml human serum albumin (HSA, Statens Seruminstitut, Denmark), 0.23mM pyruvate (Sigma, Cat. No S-8636), 2 mM glutamine (Flow Cat. No. 16-801), 100 IU/ml penicillin and 100 $\mu$g/ml streptomycin (Flow, Cat No. 16-700). This medium was supplemented with 3 mM hypoxanthine (Sigma Cat. No. H-9377) and designated Hx-medium. The oocytes were rinsed three times in Hx-medium and oocytes of uniform size were divided into groups of CEO and NO. CEO and NO were cultured in 4-well multidishes (Nunclon, Denmark) in which each well contained 0.4 ml of Hx-medium and the compound to be tested in a concentration of 10 $\mu$M. One control well (i.e., 35-45 oocytes cultured in identical medium with no addition of test compound) was always cultured simultaneously with 3 test wells (35-45 oocytes per well supplemented with test compound). The oocytes were cultured in a humidified atmosphere of 5% $CO_2$ in air for 24 hours at 37°C. By the end of the culture period, the number of oocytes with GV, GVB and polar bodies (hereinafter designated PB), respectively, were counted using a stereo microscope (Wildt, Leica MZ 12). The percentage of GVB, defined as percentage of oocytes undergoing GVB per total number of oocytes in that well, was calculated as:

$$\%\ GVB = ((\text{number of GVB} + \text{number of PB}) /\ \text{total number of oocytes}) \times 100.$$

LEGEND TO FIGURES

**Figure 1.** Schematic diagram of the study.

**Figure 2.** Illustrates the degree of cumulus-corona cells expansion originating from pre- or post-hCG treated patients at 0 hour followed by culture in control for 24 and 48 hours. On each column are marked the proportion of different COC types.

* denotes difference for types I and II COC from post-hCG ($p < 0.001$).
* * denotes difference for types II and IV COC from post-hCG ($p < 0.05$).

**Figure 3.** Illustrates the degree of cumulus-corona cells expansion originating from pre- or post-hCG treated patients at 0 hour followed by culture in DDMP for 24 and 48 hours. On each column are marked the proportion of different COC types.

* denotes difference for types I and II COC from post-hCG ($p < 0.05$).
** denotes difference for type I COC from post-hCG ($p < 0.01$).
*** denotes difference for types 11 and IV COC from post-hCG ($p < 0.05$).

**Figure 4.** Percentual proportion of nuclear maturation stages at 0, 24 and 48 hours culture of COCs collected from pre-hCG treated patients. N/A = oocytes not analyzed.

* denotes significant difference from controls ($p < 0.001$) by $\chi 2$ test.

**Figure 5.** Percentual proportion of nuclear maturation stages at 0, 24 and 48 hours culture of COCs collected from post-hCG treated patients. N/A = oocytes not analyzed.

* denotes significant difference from controls ($p < 0.001$) by $\chi 2$ test.

**Figure 6.** COC fixed immediately after retrieval. (a) Light microscopy of COC at time of retrieval. Note round cumulus-cells surrounding the oocyte. (x400). (b) Part of oocyte nucleus with intact nuclear membrane. Note the presence of the karyosphere surrounding the dark compact nucleolus. The cytoplasm surrounding the nucleus is covered with aggregates of mitochondria and vesicles of SER. (x3000). (c) Cortex of oocyte with clumps of mitochondria forming aggregates with SER (arrow). Note the areas devoid of organelles. Microvilli are projected from the oolemma to the inner cortex of ZP (*). Cortical granules (CG) dispersed throughout the cytoplasm and there is the start of formation of a single layer under the oolemma (arrowheads). (x3000). (d) Adherence between cumulus cells connected by gap junction (arrow). (x12000).
N= nucleolus; K= karyosphere; SER= smooth endoplasmic reticulum; MT= mitochondria;

**Figure 7.** COC matured *in vitro* for 24 hours without inhibitor. (a) Light microscopy section with numerous elongated cumulus-cells. Note organelles dispersed throughout cytoplasm. (x400). (b) Cortex of polar body extruded oocyte with dispersion of organelles throughout cytoplasm. Note complexes of SER with peripheral mitochondria (*) (x3000). CG form one to two layers under oolemma (arrowhead). (c) Cumulus cells are elongated and retracted from the zona pellucida. The nuclei of the cells are positioned at the periphery at the opposite side of the elongation of the cytoplasm. Note the occasionally degenerating nucleus (arrow). (x1100). mc= microvilli.

**Figure 8.** COC arrested *in vitro* for 24 hours by DDMP. (a) Light microscopy section with cumulus-cells starting to retract from zona but their nucleus is more centralised in the cell. Note organelles distributed in clumps in immature oocyte (GV not shown). (x400). (b) The nucleus is centrally located involved by an intact and smooth nuclear membrane (arrowhead). The nucleolus situated close to nuclear membrane is surrounded by a karyosphere. The mitochondriae are surrounding the GV intermingled with vesicles. (x 1100). (c) and (d) The cortex of oocyte is devoid of organelles and mitochondriae are distributed forming clumps with SER. (d) Cumulus cells are retracted from zona. Lipid droplets are observed (arrowheads). (c = x3000; d = x1100).
GV=germinal vesicle; N= nucleolus; K= karyosphere; MT= mitochondria; ZP= zona pellucida.

**Figure 9.** COC matured *in vitro* for 48 hours without inhibitor. (a) The first polar body is separated from the oocyte lying in a distended perivitelline space. Oocyte chromosomes appear as clumps of dense chromatin and lie on the equator of the metaphase spindle (arrow). Mitochondriae and SER are dispersed throughout the ooplasm, and one layer of CG appears beneath the oolemma (arrowhead). (x3000). (b) Cortex of PB extruded oocyte with two-three layers of CG and some vesicles of SER of which some are swollen (*). (x7000). PVS=perivitelline space; PB= polar body.

**Figure 10.** COC arrested *in vitro* for 48 hours in the presence of DDMP. (a) Oocyte with centrally located nucleus with presence of karyosphere around the nucleoli. Note a still intact nuclear membrane (arrow). Organelles are intermingled with vesicles of SER, which are mainly localised around nucleus but start to dissociate from their aggregates. (x3000). (b) Oocyte at GV-stage has a more homogeneous distribution of organelles in the cytoplasm.

Beneath the oolemma cortical granules are forming a single layer (arrows). (x 1100).GV=germinal vesicle; N= nucleolus; K= karyosphere.

**Figure 11.** COC arrested *in vitro* for 72 hours in presence of DDMP showing degenerative aspect. (a) Light microscopy section showing numerous increased vesiculation of SER around GV. (x1000). (b) Oocyte with signs of degeneration with increased vacuolisation of SER and mitochondria are also degenerated (arrowheads). Note lack of microvilli on oocyte cortex (arrow) and a more homogeneous zona (ZP). (x 7000). SER= smooth endoplasmic reticulum. N= nucleolus; K= karyosphere.

**Figure 12.** COC matured *in vitro* for 48 hours without inhibitor. Single sections of confocal images representing the microfilaments (actin) in M II oocyte. In (a) and (b) left and right are the same oocyte at different levels of the M II plate with chromosomal disarrangement.

**Figure 13.** COC cultured for 30h after arrest *in vitro* for 72 hours. Single sections of confocal images representing the microfilaments (actin) in M II oocyte. In (a) and (b) left and right are the same oocyte at different levels. The level of the first polar body F-actin displays a more predominant red staining than at the level of the metaphase plate.

## Claims

**1.** A method for in vitro synchronisation of nuclear and cytoplasmatic maturation of GV oocytes from domestic animals or from primates comprising the steps of:

a. culturing one or more GV oocytes or MI oocytes from domestic animals or from humans in a first culture medium, the culture medium comprising a pyridazinone derivative of the general formula I

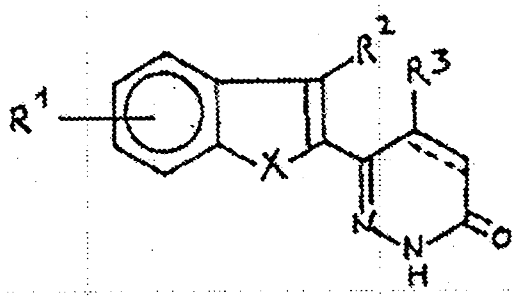

I

wherein $R^1$ represents one to four substituents, which may be the same or different and are selected from H, OH, halogen, $NO_2$ unsubstituted or C1-C4 alkyl substituted amino, C1-C4 alkyl, C1-C4 halogen substituted alkyl, O-ALK-$NR^4R^5$, C1-C4 alkoxy, whereby two substituents taken together may also represent a methylenedioxy group; $R^2$ and $R^3$ represent independently H or C1-C4 alkyl; $R^4$ and $R^5$ represent independently H or C1-C4 alkyl, or form together with the nitrogen a 5- or 6-membered ring; X represents S or O; the dotted line represents an optional bond; and their pharmaceutically acceptable salts, the culturing taking place for a time period sufficient for cytoplasmatic maturation to progress or to allow for normal working hours during the IVF/ICSI procedure;

b. washing the oocytes of step (a) to remove the compound of the general formula I or decrease the amount thereof;

c. culturing the washed oocytes of step (b) in a second culture medium for a time period sufficient for nuclear maturation to be completed up to metaphase II to a desired frequency.

**2.** A method, according to claim 1, wherein human oocytes are used.

**3.** A method, according to any one of the preceding claims, wherein the oocytes used for culturing in the first medium are oocytes which have been stored in an oocyte collection medium being a $CO_2$-independent medium containing a compound of the general formula I.

**4.** A method, according to the preceding claim, wherein the oocyte has been placed in said oocyte collection medium not later than 4 hours, preferably not later than 2 hours, even more preferred not later than 1 hour, and even more

preferred not later than ½ an hour, after said oocyte has been removed from the woman.

5. A method, according to any one of the preceding claims, wherein the culturing according to step (a) takes place for a time period sufficient for cytoplasmatic maturation to be improved.

6. A method, according to the preceding claim, wherein the culturing according to step (a) takes place for a time period sufficient for cytoplasmatic maturation to be completed.

7. A method, according to any one of the preceding claims, wherein the oocytes cultured in step (a) are GV oocytes.

8. A method, according to any one of the preceding claims, wherein the first culture medium contains EGF, GH, gonadotropin combinations, promoters of glutathione synthesis, meiosis activating compounds, combinations of activin and inhibin, the analogues of the foregoing or combinations of the aforementioned compounds.

9. A method, according to any one of the preceding claims, wherein the compound of the general formula I is DDMP.

10. A method, according to any one of the preceding claims, wherein the culturing time in step (a) is at least about 4 hours.

11. A method, according to any one of the preceding claims, wherein the culturing time is at least about 8 hours.

12. A method, according to any one of the preceding claims, wherein the culturing time is at least about 12 hours.

13. A method, according to any one of the preceding claims, wherein the culturing time is at least about 24 hours.

14. A method, according to any one of the preceding claims, wherein the culturing time is at least about 48 hours.

15. A method, according to any one of the preceding claims, wherein the culturing time is at least about 72 hours.

16. A method, according to any one of the preceding claims, wherein the oocytes used in step (a) originate from follicles which were aspirated from the animal/human (cycling animal/human or not) before any hormone treatment that could effect either folliculogenesis and/or oogenesis.

17. A method, according to the preceding claim, wherein the hormone treatment of the woman from which the oocytes originates had been performed with a steroid, a gonadotrophin, a GnRHanalogue, clomiphene citrate, tamoxiphen, an insulin sensitiser (e g. metformin), an aromatase inhibitor or any of the preceding drugs alone or combined.

18. A method, according to any one of the preceding claims, wherein the oocytes originate from follicles which were aspirated from the animal after any hormone or drug treatment that effects upon folliculogenesis and/or oogenesis.

19. A method, according to the preceding claim, wherein the hormone or drug treatment had been performed with a gonadotropin, a GnRHanalogue, clompihene citrate, tamoxiphen, LH, HCG, a steroid or a steroid-like substance, an LHRH analogue, an aromatase inhibitor, an insulin sensitizing agent, and any analogue or combination thereof.

20. A method, according to any one of the two preceding claims, wherein the hormone used had been hCG, LH or any compound which might induce similar effects.

21. A method according to any one of the preceding claims wherein the washing of the oocytes from step (a) is performed so that the concentration of the compound of the general formula I after the washing out of said compound in the second medium is less than about 10%, preferably less than about 5 %, more preferred less than about 1% of the concentration of said compound in the first medium.

22. A method according to any one of the preceding claims wherein the first culture medium is minimally covered by a thin layer of an oil so that the ratio between the oily phase and the aqueous phase is less that about 2:10 (vol/vol), preferably less that about 1:10 (vol/vol).

23. A method according to any one of the preceding claims whereby the oocyte collection medium is covered by oil.

24. A method according to any one of the preceding claims where, in step (a), there are cumulus cells around the oocyte.

**25.** A method according to any one of the preceding claims where, in step (b), washing the oocytes of step (a) to remove the compound of the general formula I or decrease the amount thereof is performed so that the remaining amount of said compound, if any, has no substantial effect on the arresting of the oocytes.

**26.** A method according to any one of the preceding claims where, in step (c), there are no or substantially no cumulus cells around the oocyte.

**27.** A method, according to any one of the preceding claims, wherein, in step (c), the nuclear maturation is stimulated by adding HCG and/or EGF and/or a MAS compound to the second culture medium.

**28.** A method, according to any one of the preceding claims, wherein, in step (c), the desired frequency is at least about 30 %, more preferred at least about 50 %, even more preferred at least about 70 %, and preferably at least about 90 %.

**29.** A method according to any one of the preceding claims whereby, after step (c), the oocytes are fertilized with sperm, preferably using ICSI.

**Patentansprüche**

**1.** Verfahren zur in-vitro-Synchronisation der Kern- und Zytoplasmareifung von GV-Oozyten von Haustieren oder Primaten, umfassend die Schritte:

a. Kultivieren von einem oder mehreren GV-Oozyten oder MI-Oozyten von Haustieren oder Menschen in einem ersten Kulturmedium, wobei das Kulturmedium ein Pyridazinonderivat der allgemeinen Formel 1

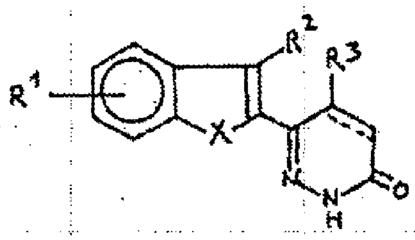

I

wobei $R^1$ ein bis vier Substituenten darstellt, die gleich oder verschieden sein können und ausgewählt sind aus H, OH, Halogen, $NO_2$, unsubstituiertem oder C1-C4-alkylsubstituiertem Amino, C1-C4-Alkyl, C1-C4-halogensubstituiertem Alkyl, O-ALK-$NR^4R^5$, C1-C4-Alkoxy, wobei zwei Substituenten zusammen auch eine Methylendioxygruppe darstellen können; $R^2$ und $R^3$ unabhängig H oder C1-C4-Alkyl darstellen; $R^4$ und $R^5$ unabhängig H oder C1-C4-Alkyl darstellen oder zusammen mit den Stickstoff einen 5- oder 6-gliedrigen Ring bilden; X S oder O darstellt; die gestrichelte Linie eine optionale Bindung darstellt, und deren pharmazeutisch verträglichen Salze umfasst, wobei das Kultivieren für eine Zeitdauer stattfindet, die für den Verlauf einer Zytoplasmareifung oder zum Ermöglichen von normalen Arbeitsstunden während des IVF/ICSI-Verfharens ausreichend ist;
b. Waschen der Oozyten von Schritt (a) zum Entfernen der Verbindung der allgemeinen Formel I oder Vermindern der Menge davon;
c. Kultivieren der gewaschenen Oozyten von Schritt (b) in einem zweiten Kulturmedium für eine Zeitdauer, die für die Vollständigkeit der Kernreifung bis zur Metaphase II mit einer gewünschten Häufigkeit ausreichend ist.

**2.** Verfahren nach Anspruch 1, wobei menschliche Oozyten verwendet werden.

**3.** Verfahren nach einem der vorangehenden Ansprüche, wobei es sich bei den zum Kultivieren im ersten Medium verwendeten Oozyten um Oozyten handelt, die in einem Oozytensammelmedium, bei welchem es sich um ein $CO_2$-unabhängiges Medium, enthaltend eine Verbindung der allgemeinen Formel I, handelt, aufbewahrt worden sind.

**4.** Verfahren nach dem vorangehenden Anspruch, wobei der Oozyt nicht später als 4 Stunden, vorzugsweise nicht später als 2 Stunden, noch stärker bevorzugt nicht später als 1 Stunde und noch stärker bevorzugt nicht später als ½ Stunde nach dem Entfernen des Oozyten aus der Frau in das Oozytensammelmedium gegeben worden ist.

5. Verfahren nach einem der vorangehenden Ansprüche, wobei das Kultivieren gemäß Schritt (a) für eine Zeitdauer stattfindet, die für eine Verbesserung der Zytoplasmareifung ausreichend ist.

6. Verfahren nach dem vorangehenden Anspruch, wobei das Kultivieren gemäß Schritt (a) für eine Zeitdauer stattfindet, die zur Vollständigkeit der Zytoplasmareifung ausreichend ist.

7. Verfahren nach einem der vorangehenden Ansprüche, wobei es sich bei den in Schritt (a) kultivierten Oozyten um GV-Oozyten handelt.

8. Verfahren nach einem der vorangehenden Ansprüche, wobei das erste Kulturmedium EGF, GH, Gonadotropinkombinationen, Verstärker für die Glutathionsynthese, meioseaktivierende Verbindungen, Kombinationen von Activin und Inhibin, die Analoga von Vorstehendem oder Kombinationen der vorstehend erwähnten Verbindungen handelt.

9. Verfahren nach einem der vorangehenden Ansprüche, wobei es sich bei der Verbindung der allgemeinen Formel I um DDMP handelt.

10. Verfahren nach einem der vorangehenden Ansprüche, wobei die Kultivierungszeit in Schritt (a) mindestens etwa 4 Stunden beträgt.

11. Verfahren nach einem der vorangehenden Ansprüche, wobei die Kultivierungszeit mindestens etwa 8 Stunden beträgt.

12. Verfahren nach einem der vorangehenden Ansprüche, wobei die Kultivierungszeit mindestens etwa 12 Stunden beträgt.

13. Verfahren nach einem der vorangehenden Ansprüche, wobei die Kultivierungszeit mindestens etwa 24 Stunden beträgt.

14. Verfahren nach einem der vorangehenden Ansprüche, wobei die Kultivierungszeit mindestens etwa 48 Stunden beträgt.

15. Verfahren nach einem der vorangehenden Ansprüche, wobei die Kultivierungszeit mindestens etwa 72 Stunden beträgt.

16. Verfahren nach einem der vorangehenden Ansprüche, wobei die in Schritt (a) verwendeten Oozyten von Follikel stammen, die von dem Tier/Menschen (periodisierenden Tier/periodisierenden Menschen oder nicht) vor jeglicher Hormonbehandlung, die entweder auf die Follikulogenese und/oder Oogenese einwirkt, abgesaugt wurden.

17. Verfahren nach dem vorangehenden Anspruch, wobei die Hormonbehandlung der Frau, von welcher die Oozyten stammen, mit einem Steroid, einem Gonadotropin, einem GnRH-Analogon, Clomiphencitrat, Tamoxiphen, einem Insulinsensibilisator (z.B. Metformin), einem Aromatasehemmer oder beliebigen der vorangehenden Arzneistoffe allein oder kombiniert durchgeführt worden ist.

18. Verfahren nach einem der vorangehenden Ansprüche, wobei die Oozyten von Follikel stammen, die vom Tier nach jeglicher Hormon- oder Arzneistoffbehandlung, die auf die Follikulogenese und/oder Oogenese einwirkt, abgesaugt wurden.

19. Verfahren nach dem vorangehenden Anspruch, wobei die Hormon- oder Arzneistoffbehandlung mit einem Gonadotropin, einem GnRH-Analogon, Clomiphencitrat, Tamoxipen, LH, HCG, einem Steroid oder einer steroidartigen Substanz, einem LHRH-Analogon, einem Aromatasehemmer, einem Insulinsensibilisator und einem beliebigen Analogon oder einer beliebigen Kombination davon durchgeführt worden ist.

20. Verfahren nach einem der beiden vorangehenden Ansprüche, wobei das verwendete Hormon hCG, LH oder eine beliebige Verbindung, die ähnliche Wirkungen herbeiführen könnte, ist.

21. Verfahren nach einem der vorangehenden Ansprüche, wobei das Waschen der Oozyten von Schritt (a) derart durchgeführt wird, dass die Konzentration der Verbindung der allgemeinen Formel I nach dem Herauswaschen der Verbindung im zweiten Medium weniger als etwa 10%, vorzugsweise weniger als etwa 5%, stärker bevorzugt

weniger als etwa 1 % der Konzentration der Verbindung im ersten Medium beträgt.

**22.** Verfahren nach einem der vorangehenden Ansprüche, wobei das erste Kulturmedium derart minimal durch eine dünne Schicht aus Öl bedeckt ist, dass das Verhältnis zwischen der öligen Phase und der wässrigen Phase weniger als etwa 2:10 Vol.-%, vorzugsweise weniger als etwa 1:10 Vol.-% beträgt.

**23.** Verfahren nach einem der vorangehenden Ansprüche, wobei das Oozytensammelmedium mit Öl bedeckt ist.

**24.** Verfahren nach einem der vorangehenden Ansprüche, wobei in Schritt (a) Kumuluszellen um den Oozyten vorliegen.

**25.** Verfahren nach einem der vorangehenden Ansprüche, wobei in Schritt (b) das Waschen der Oozyten von Schritt (a) zum Entfernen der Verbindung der allgemeinen Formel I oder Vermindern der Menge davon derart durchgeführt wird, dass die Restmenge der Verbindung, falls überhaupt, im Wesentlichen keine Wirkung auf die Hemmung der Oozyten aufweist.

**26.** Verfahren nach einem der vorangehenden Ansprüche, wobei in Schritt (c) keine oder im Wesentlichen keine Kumuluszellen um den Oozyten vorliegen.

**27.** Verfahren nach einem der vorangehenden Ansprüche, wobei in Schritt (c) die Kernreifung durch Zugabe von HCG und/oder EGF und/oder einer MAS-Verbindung zu dem zweiten Kulturmedium stimuliert wird.

**28.** Verfahren nach einem der vorangehenden Ansprüche, wobei in Schritt (c) die gewünschte Häufigkeit mindestens etwa 30%, stärker bevorzugt mindestens etwa 50%, noch stärker bevorzugt mindestens etwa 70% und vorzugsweise mindestens etwa 90% beträgt.

**29.** Verfahren nach einem der vorangehenden Ansprüche, wobei nach Schritt (c) die Oozyten mit Sperma vorzugsweise unter Verwendung von ICSI befruchtet werden.

## Revendications

**1.** Procédé pour la synchronisation in vitro de la maturation nucléaire et cytoplasmique d'ovocytes à vésicules germinatives (GV) provenant d'animaux domestiques ou de primates, comprenant les étapes consistant :

a. à cultiver un ou plusieurs ovocytes GV ou ovocytes MI (en métaphase I) provenant d'animaux domestiques ou provenant d'humains dans un premier milieu de culture, le milieu de culture comprenant un dérivé de pyridazinone de formule générale I

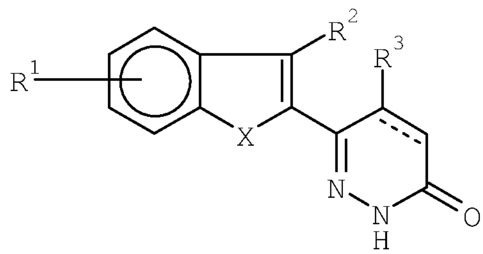

I

dans laquelle $R^1$ représente un à quatre substituants, qui peuvent être identiques ou différents et sont choisis parmi H, OH, les groupes halogéno, $NO_2$, amino non substitué ou à substitution alkyle en $C_1$-$C_4$, alkyle en $C_1$-$C_4$, alkyle halogéné en $C_1$-$C_4$, O-ALK-$NR^4R^5$, alcoxy en $C_1$-$C_4$, où deux substituants, pris ensemble, peuvent aussi représenter un groupe méthylènedioxy ; $R^2$ et $R^3$ représentent d'une manière indépendante chacun H ou un groupe alkyle en $C_1$-$C_4$ ; $R^4$ et $R^5$ représentent d'une manière indépendante chacun H ou un groupe alkyle en $C_1$-$C_4$, ou encore avec l'azote forment un noyau à 5 ou 6 chaînons ; X représente S ou O ; la ligne en tirets représente une liaison en option ; et leurs sels acceptables d'un point de vue pharmaceutique, la culture ayant lieu pendant un laps de temps suffisant pour faire avancer la maturation cytoplasmique ou lui permettre d'être effectué pendant les heures normales de travail pendant une intervention FIV/ICSI ;

b. à laver les ovocytes de l'étape (a) pour éliminer le composé de formule générale I ou en diminuer la quantité ;

c. à cultiver les ovocytes lavés de l'étape (b) dans un deuxième milieu de culture pendant un laps de temps suffisant pour parachever la maturation nucléaire jusqu'à la métaphase II à une fréquence souhaitée.

**2.** Procédé selon la revendication 1, dans lequel on utilise des ovocytes humains.

**3.** Procédé selon l'une quelconque des revendications précédentes, dans lequel les ovocytes utilisés pour la culture dans le premier milieu sont des ovocytes qui ont été stockés dans un milieu de collecte d'ovocytes, qui est un milieu indépendant du $CO_2$ et contenant un composé de formule générale I.

**4.** Procédé selon la revendication précédentes, dans lequel l'ovocyte a été placé dans ledit milieu de collecte d'ovocytes pas plus tard que 4 heures, de préférence pas plus tard que 2 heures, d'une manière encore plus préférée pas plus tard qu'une heure et d'une manière encore plus préférée pas plus tard qu'une demi-heure après que ledit ovocyte a été éliminé de la femme.

**5.** Procédé selon l'une quelconque des revendications précédentes, dans lequel la culture selon l'étape (a) a lieu pendant un laps de temps suffisant pour une amélioration de la maturation cytoplasmique.

**6.** Procédé selon la revendication précédente, dans lequel la culture selon l'étape (a) a lieu pendant un laps de temps suffisant pour un parachèvement de la maturation cytoplasmique.

**7.** Procédé selon l'une quelconque des revendications précédentes, dans lequel les ovocytes cultivés dans l'étape (a) sont des ovocytes GV.

**8.** Procédé selon l'une quelconque des revendications précédentes, dans lequel le premier milieu de culture contient de l'EGF, de la GH, des combinaisons de gonadotrophines, des promoteurs de la synthèse du glutathion, des composés activateurs de méiose, des combinaisons d'activine et d'inhibine, des analogues de ce qui précède, ou des combinaisons des composés mentionnés ci-dessus.

**9.** Procédé selon l'une quelconque des revendications précédentes, dans lequel le composé de formule générale I est le DDMP.

**10.** Procédé selon l'une quelconque des revendications précédentes, dans lequel la durée de la culture de l'étape (a) est d'au moins environ 4 heures.

**11.** Procédé selon l'une quelconque des revendications précédentes, dans lequel la durée de la culture est d'au moins environ 8 heures.

**12.** Procédé selon l'une quelconque des revendications précédentes, dans lequel la durée de la culture est d'au moins environ 12 heures.

**13.** Procédé selon l'une quelconque des revendications précédentes, dans lequel la durée de la culture est d'au moins environ 24 heures.

**14.** Procédé selon l'une quelconque des revendications précédentes, dans lequel la durée de la culture est d'au moins environ 48 heures.

**15.** Procédé selon l'une quelconque des revendications précédentes, dans lequel la durée de la culture est d'au moins environ 72 heures.

**16.** Procédé selon l'une quelconque des revendications précédentes, dans lequel les ovocytes utilisés dans l'étape (a) proviennent de follicules qui ont été aspirés à partir d'un humain/d'un animal (cyclage animal/humain ou non), avant tout traitement hormonal qui pourrait effectuer une folliculogenèse et/ou une ovogenèse.

**17.** Procédé selon la revendication précédente, dans lequel le traitement hormonal de la femme dont proviennent les ovocytes a été mis en oeuvre avec un stéroïde, une gonadotrophine, un analogue de la gonadolibérine GnRH, du citrate de clomiphène, du tamoxiphène, un insulino-sensibilisant (par exemple la metformine), un inhibiteur d'aromatase ou l'un quelconque des médicaments ci-dessus, seul ou en combinaison.

**18.** Procédé selon l'une quelconque des revendications précédentes, dans lequel les ovocytes proviennent de follicules qui ont été aspirés de l'animal après tout traitement hormonal ou médicamenteux agissant sur la folliculogenèse et/ou l'ovogenèse.

**19.** Procédé selon la revendication précédente, dans lequel le traitement hormonal ou médicamenteux a été mis en oeuvre avec une gonadotrophine, un analogue de la gonadolibérine GnRH, du citrate de clomiphène, du tamoxiphène, de la lutéostimuline LH, de la gonadotrophine chorionique HCG, un stéroïde ou une substance analogue à un stéroïde, un analogue de la lulibérine LHRH, un inhibiteur d'aromatase, un agent insulino-sensibilisant, et tout analogue ou combinaison de ces derniers.

**20.** Procédé selon l'une quelconque des deux revendications précédentes, dans lequel l'hormone utilisée a été la hCG, la LH ou tout composé susceptible de provoquer des effets analogues.

**21.** Procédé selon l'une quelconque des revendications précédentes, dans lequel le lavage des ovocytes de l'étape (a) est mis en oeuvre de telle sorte que la concentration du composé de formule générale I après lavage dudit composé dans le deuxième milieu soit inférieure à environ 10 %, de préférence inférieure à 5 %, d'une manière plus préférée inférieure à environ 1 % de la concentration dudit composé dans le premier milieu.

**22.** Procédé selon l'une quelconque des revendications précédentes, dans lequel le premier milieu de culture est recouvert d'une manière minimale par une couche mince d'une huile de telle sorte que le rapport entre la phase huileuse et la phase aqueuse soit inférieur à environ 2:10 (vol/vol), de préférence inférieur à environ 1:10 (vol/vol).

**23.** Procédé selon l'une quelconque des revendications précédentes, dans lequel le milieu de collecte d'ovocytes est recouvert d'une huile.

**24.** Procédé selon l'une quelconque des revendications précédentes, dans lequel, dans l'étape (a), il y a des cellules cumulus autour de l'ovocyte.

**25.** Procédé selon l'une quelconque des revendications précédentes, dans lequel, dans l'étape (b), le lavage des ovocytes de l'étape (a) pour éliminer le composé de formule générale I ou en diminuer la quantité est mis en oeuvre de telle sorte que la quantité restante de ce composé, à supposer qu'il en reste, ne présente aucun effet important sur l'arrêt des ovocytes.

**26.** Procédé selon l'une quelconque des revendications précédentes, dans lequel, dans l'étape (c), il n'y a pas ou pour ainsi dire pas de cellules cumulus autour de l'ovocyte.

**27.** Procédé selon l'une quelconque des revendications précédentes, dans lequel, dans l'étape (c), la maturation nucléaire est stimulée par addition de HCG et/ou d'EGF et/ou d'un activateur de méiose MAS au deuxième milieu de culture.

**28.** Procédé selon l'une quelconque des revendications précédentes, dans lequel, dans l'étape (c), la fréquence souhaitée est d'au moins 30 %, d'une manière plus préférée d'au moins environ 50 %, d'une manière encore plus préférée d'au moins environ 70 % et de préférence d'au moins environ 90 %.

**29.** Procédé selon l'une quelconque des revendications précédentes, dans lequel, après l'étape (c), les ovocytes sont fertilisés par du sperme, de préférence par la technique ICSI.

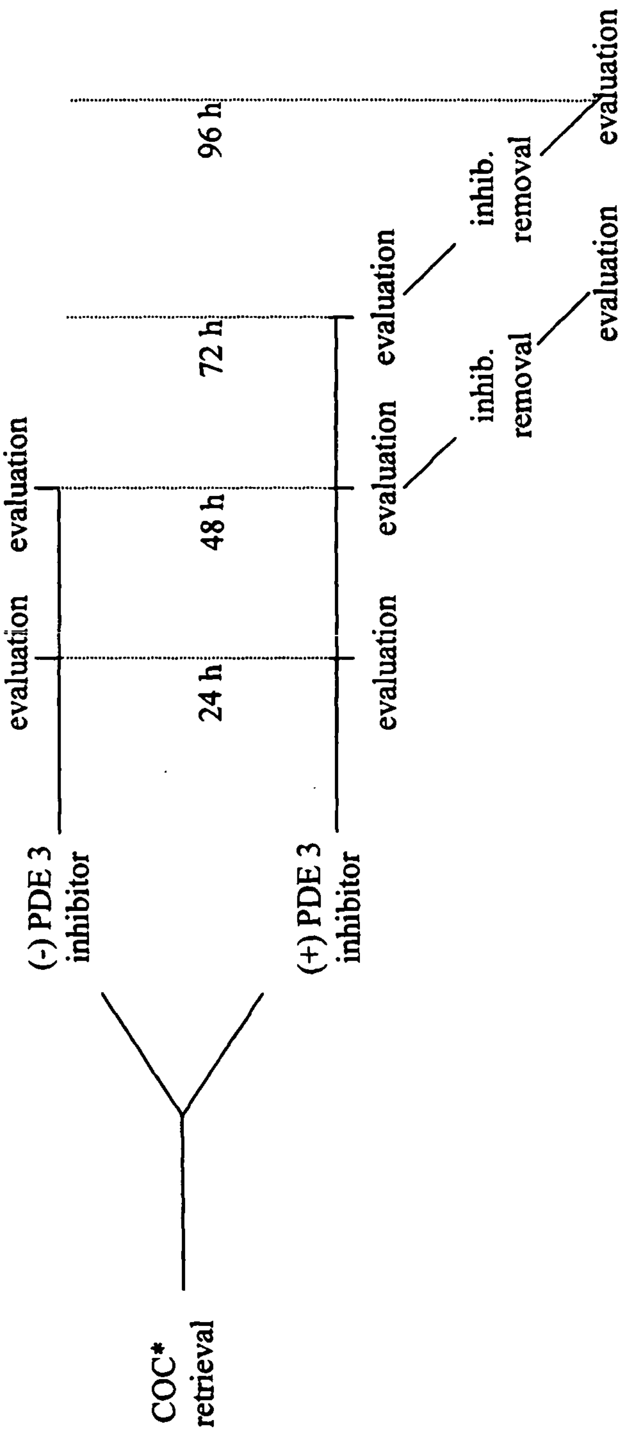
COC* retrieval
(-) PDE 3 inhibitor
(+) PDE 3 inhibitor
24 h
48 h
72 h
96 h
evaluation
evaluation
evaluation
evaluation
evaluation
evaluation
inhib. removal
evaluation
evaluation
inhib. removal
evaluation

Fig. 1

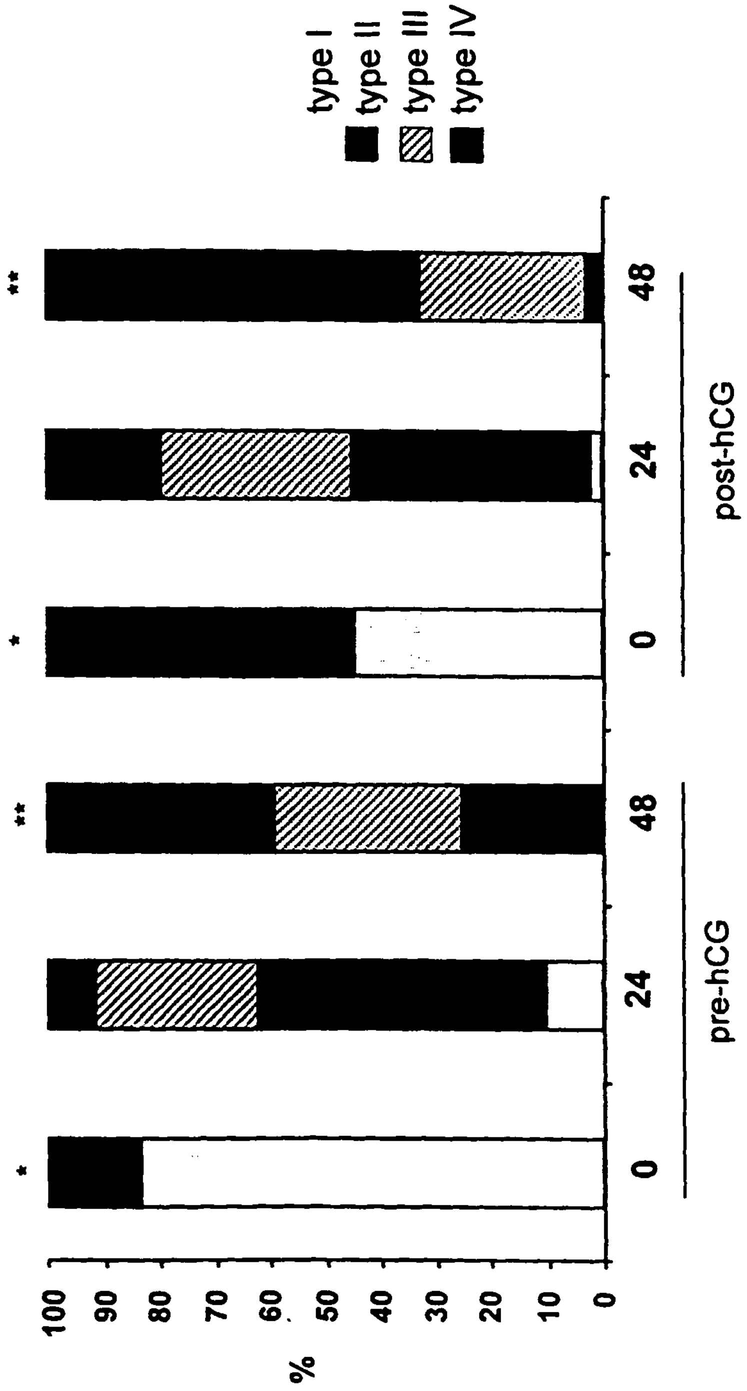
type I
type II
type III
type IV
%
100
90
80
70
60
50
40
30
20
10
0
*
**
*
**
0
24
48
0
24
48
pre-hCG
post-hCG

Fig. 2

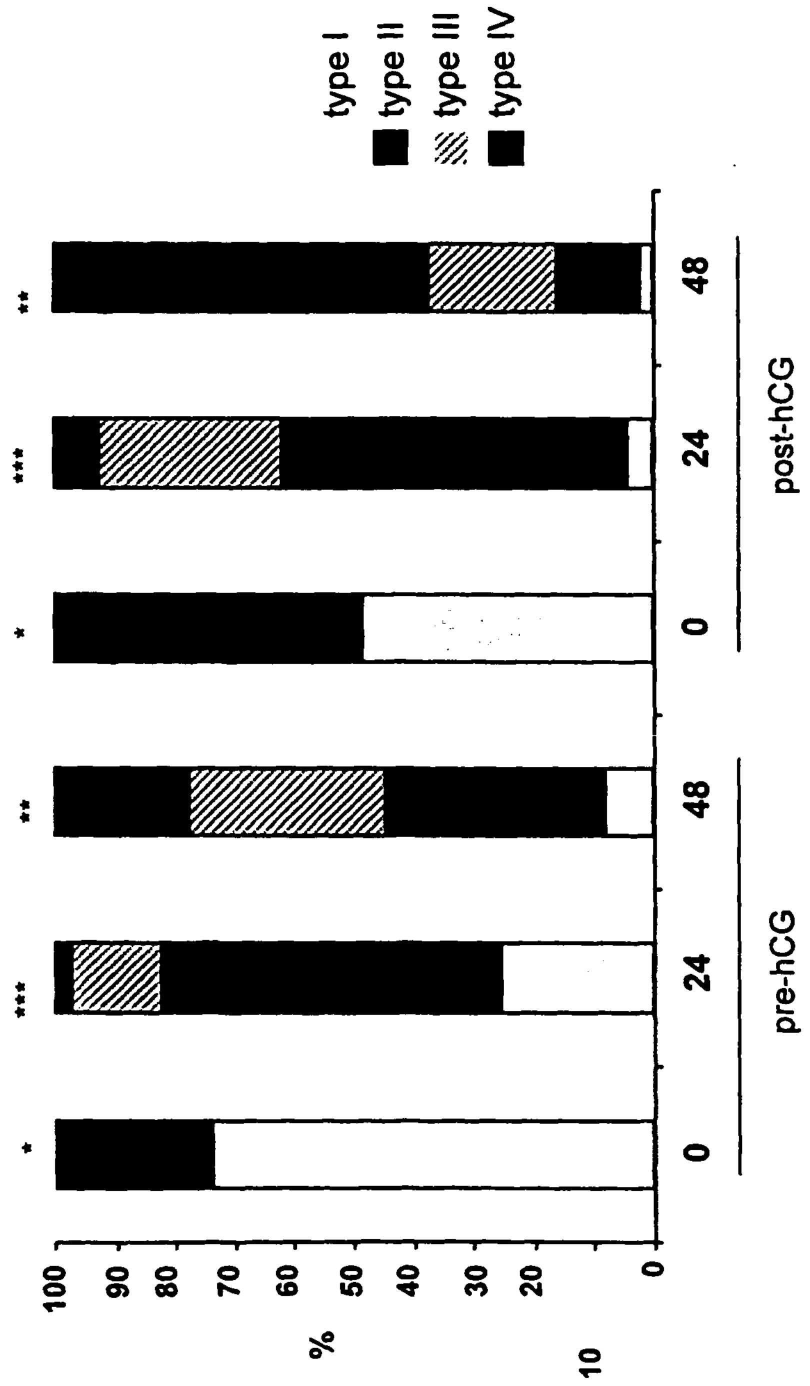
type I
type II
type III
type IV
%
100
90
80
70
60
50
40
30
20
10
0
*
***
**
0
24
48
pre-hCG
post-hCG

Fig. 3

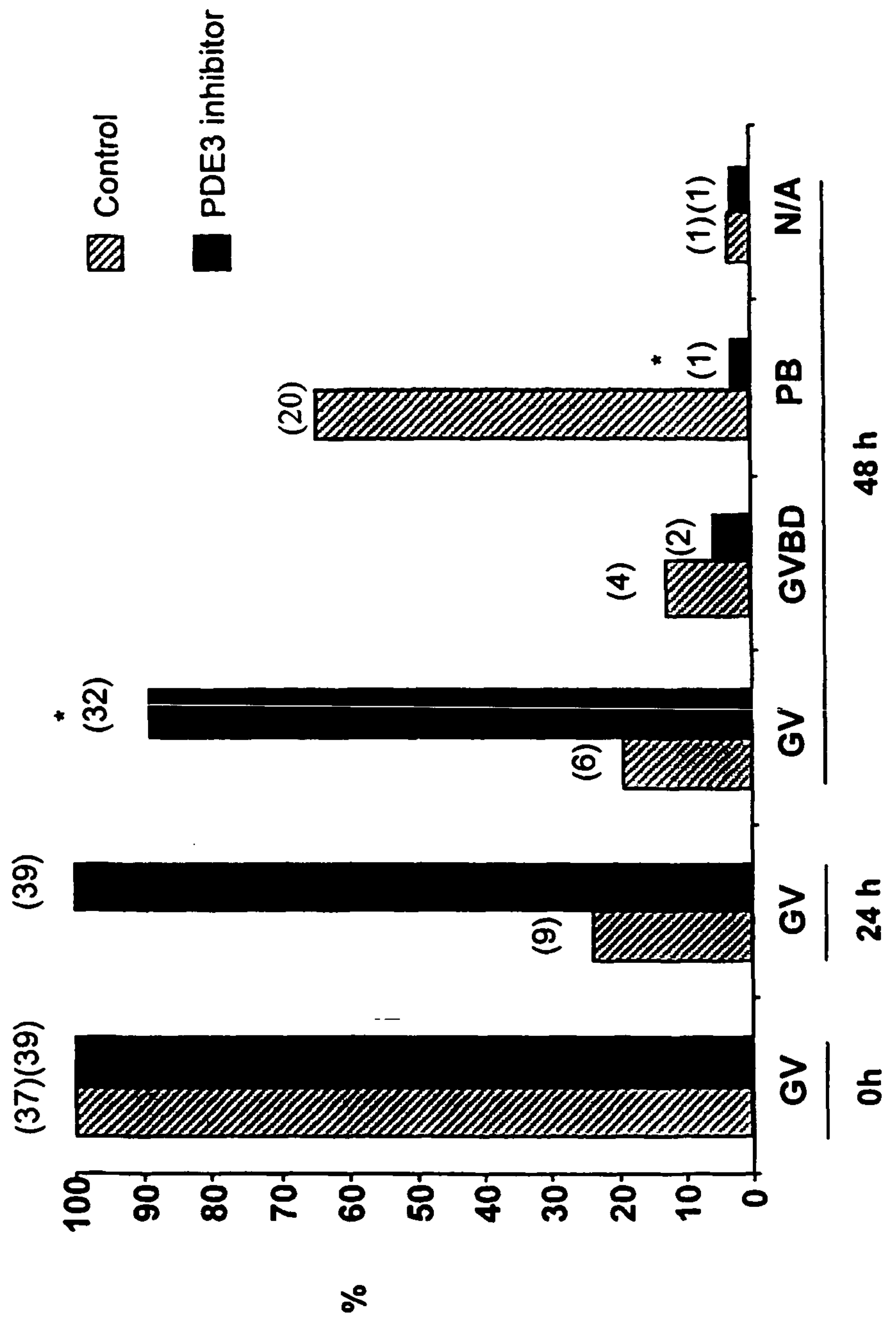
Control
PDE3 inhibitor
%
100
90
80
70
60
50
40
30
20
10
0
(37)(39)
(9)
*
(39)
(6)
*
(32)
(4)
(2)
(20)
*
(1)
(1)(1)
GV
GV
GV
GVBD
PB
N/A
0h
24 h
48 h

Fig. 4

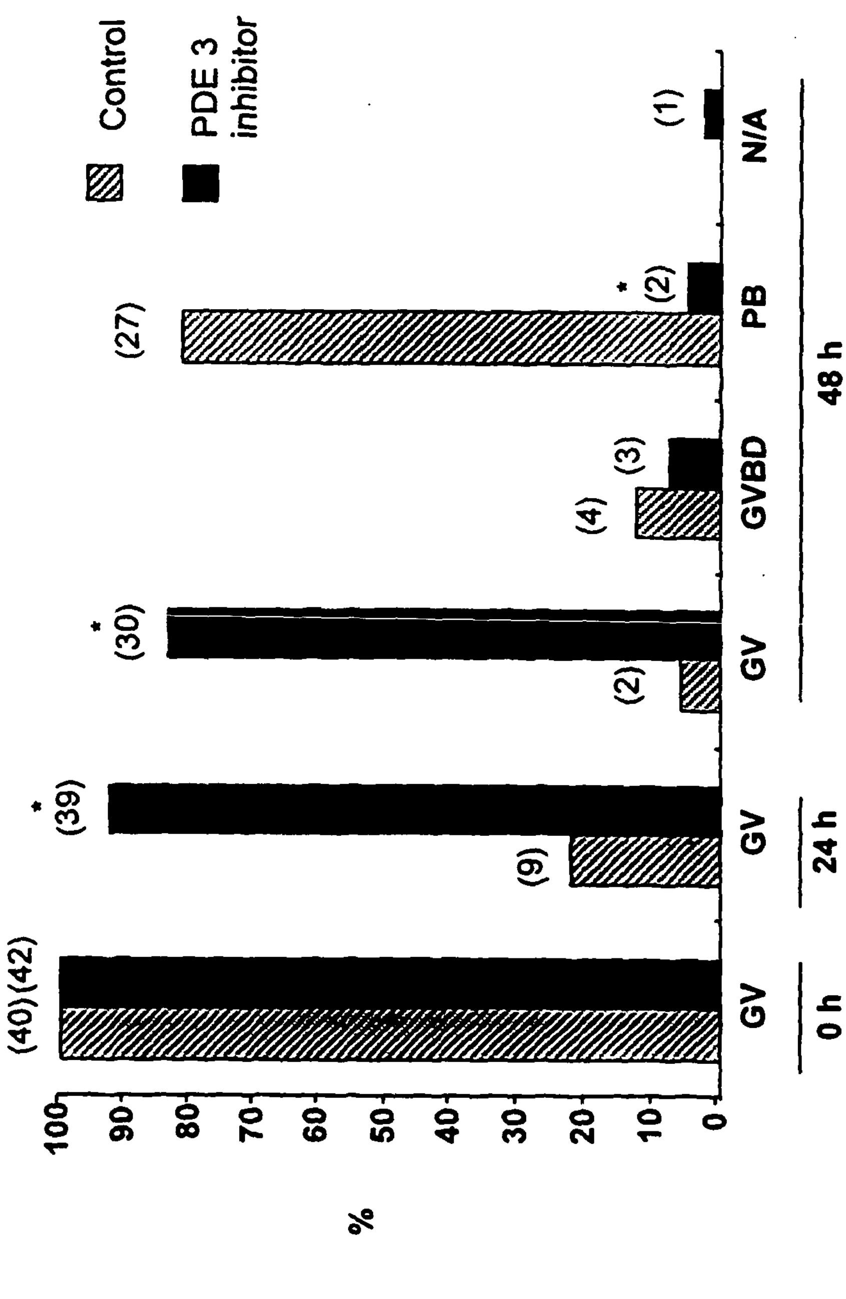
Control
PDE 3 inhibitor
%
100
90
80
70
60
50
40
30
20
10
0
(40)(42)
(9)
* (39)
(2)
* (30)
(4)
(3)
(27)
* (2)
(1)
GV
GV
GV
GVBD
PB
N/A
0 h
24 h
48 h

Fig. 5

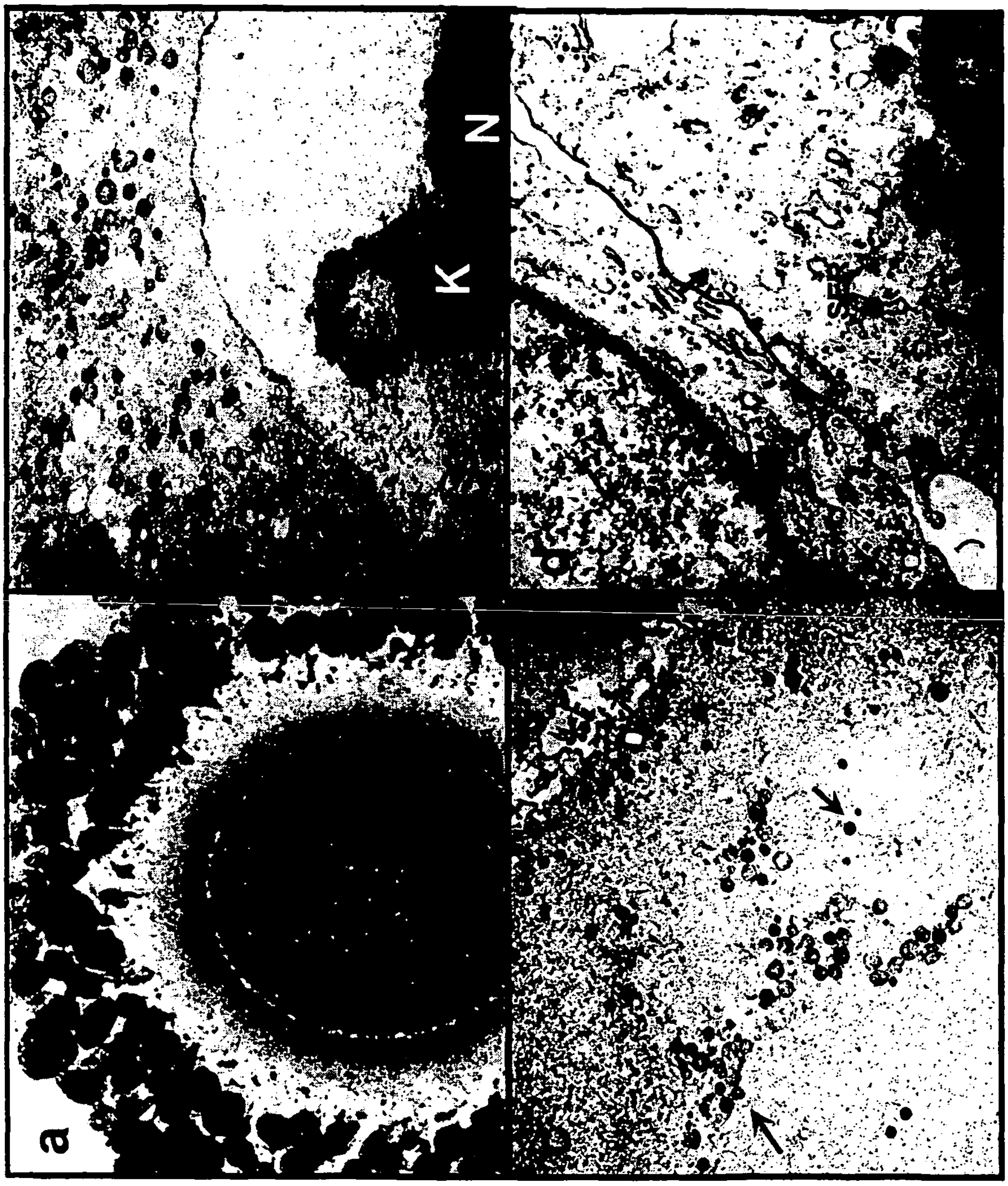


Fig. 6

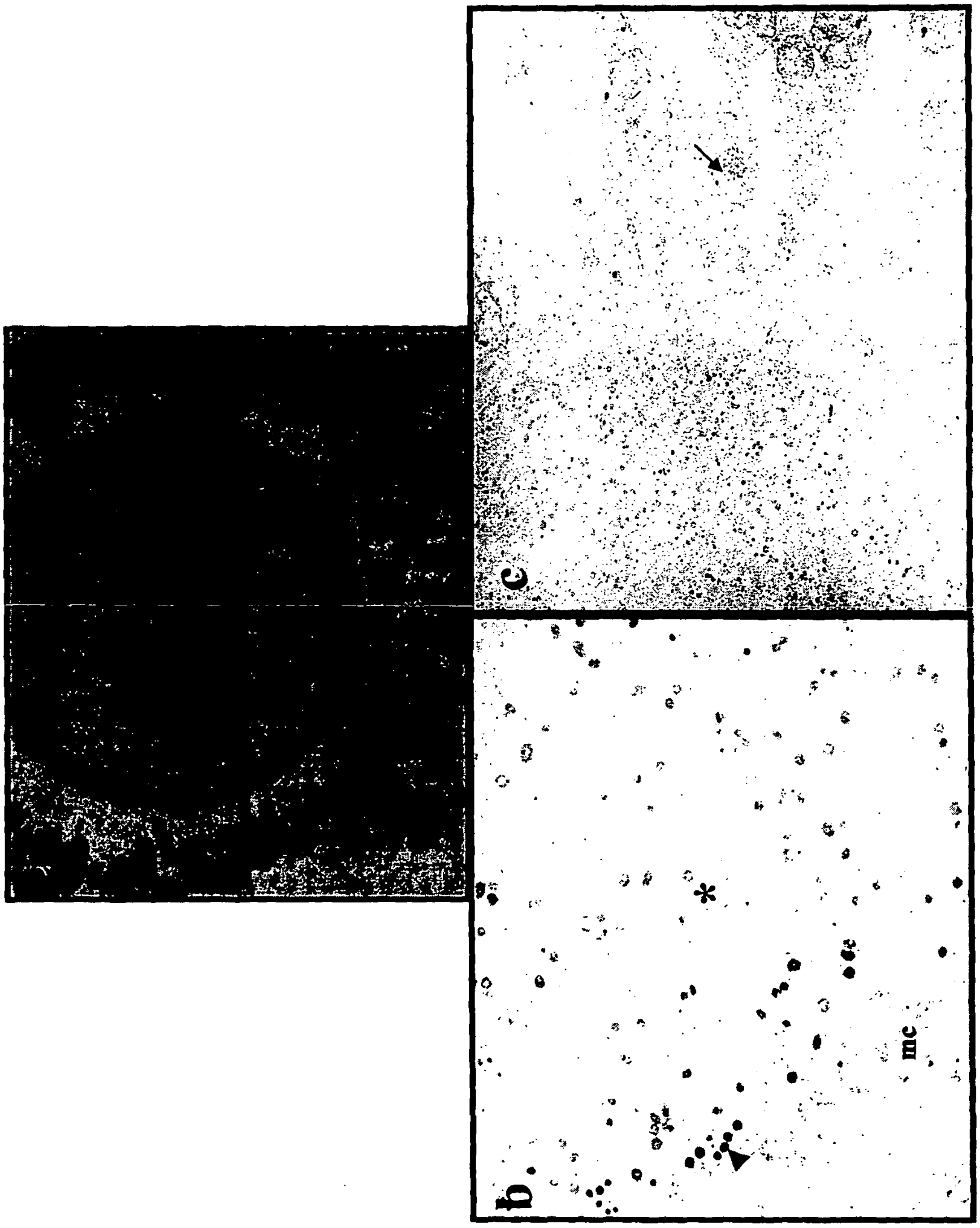


Fig. 7

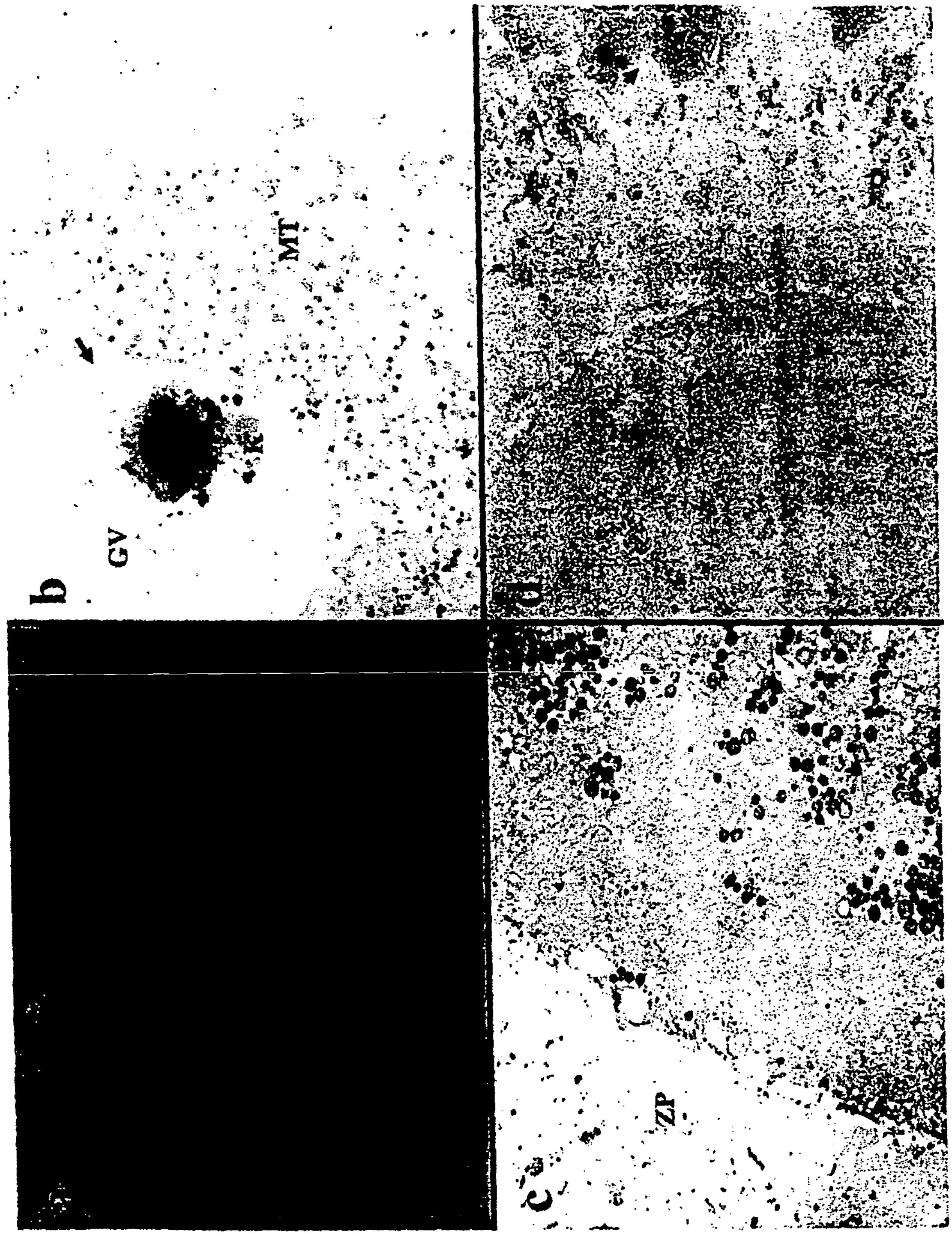


Fig. 8

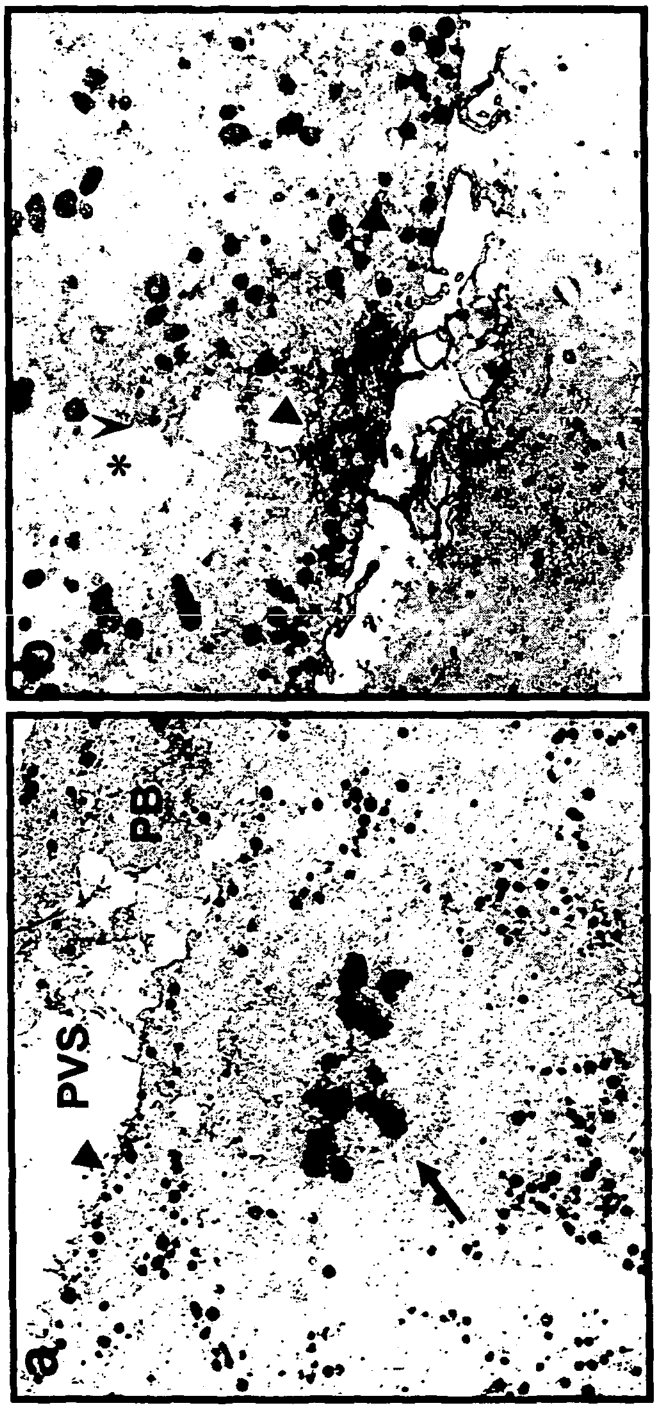


Fig. 9

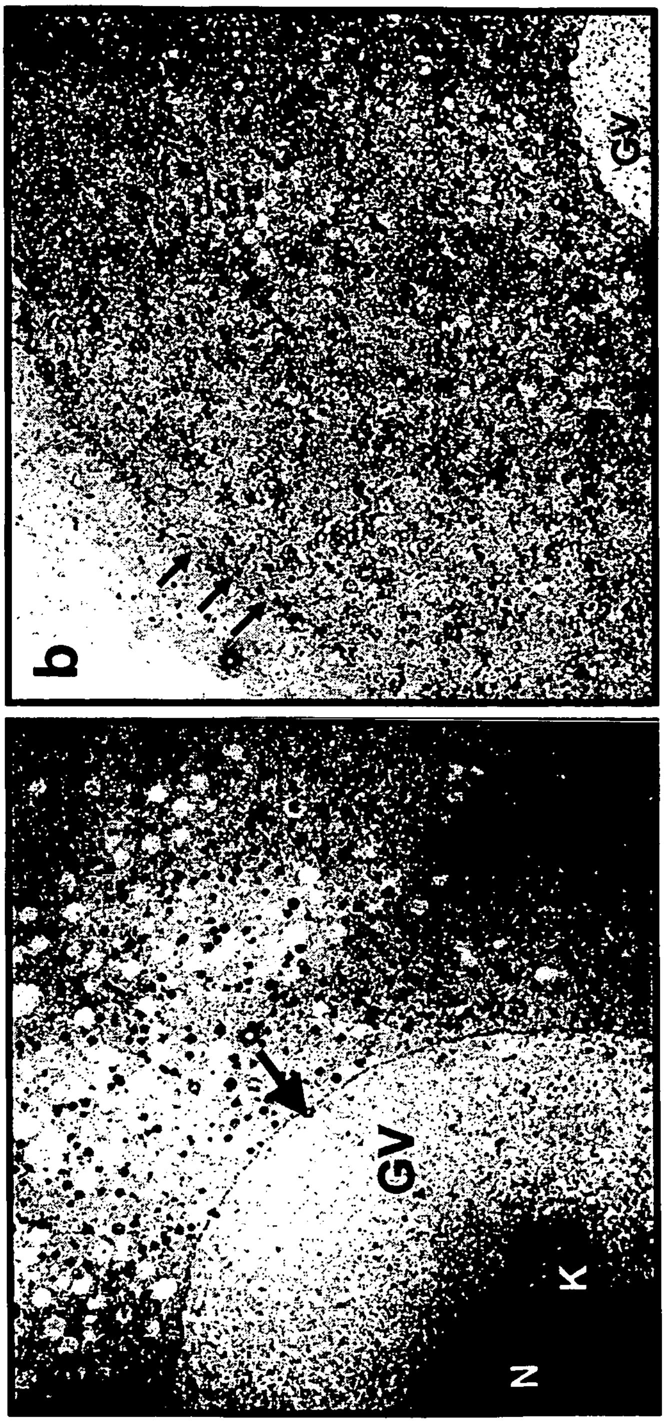


Fig. 10

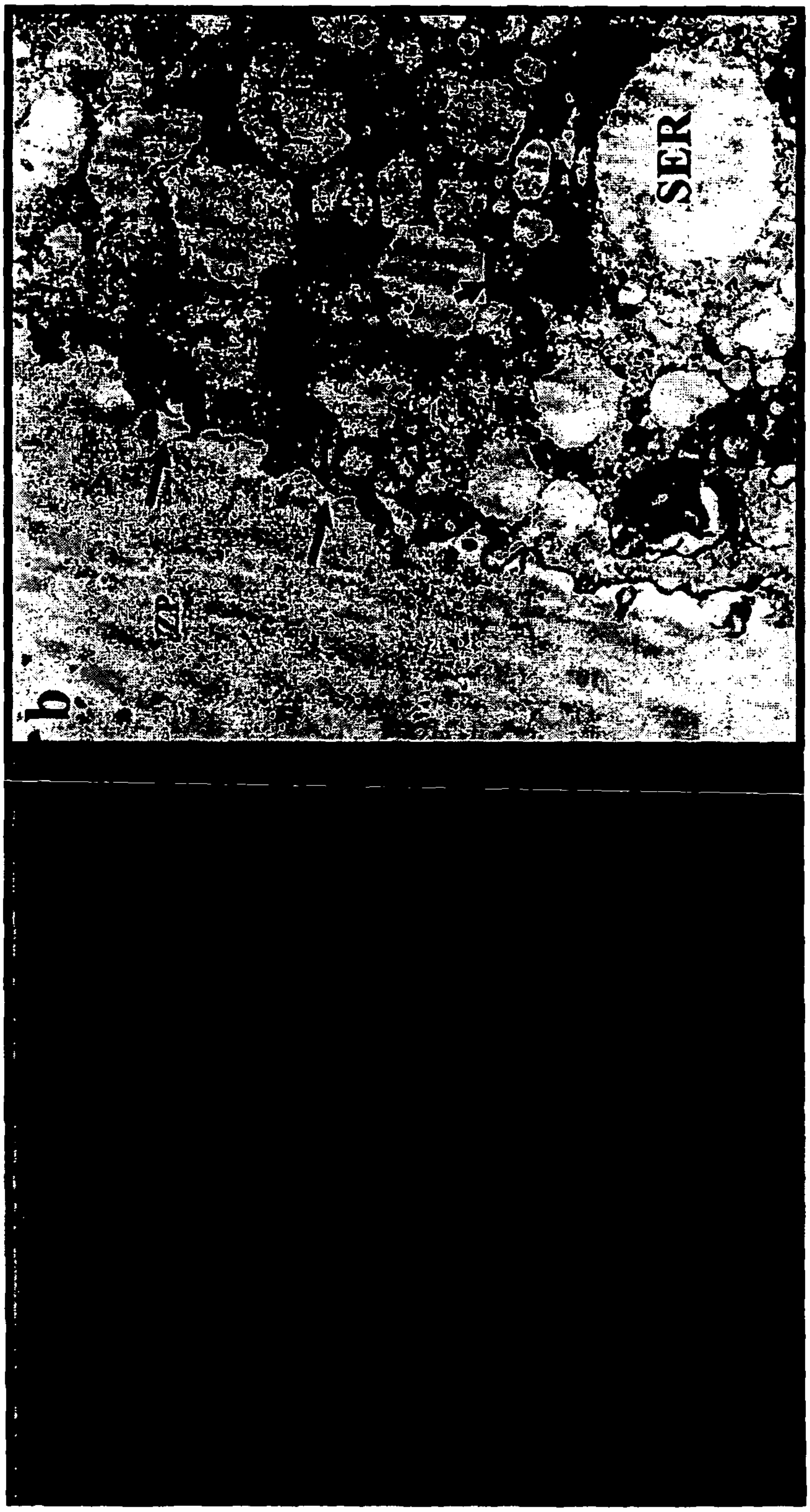


Fig. 11

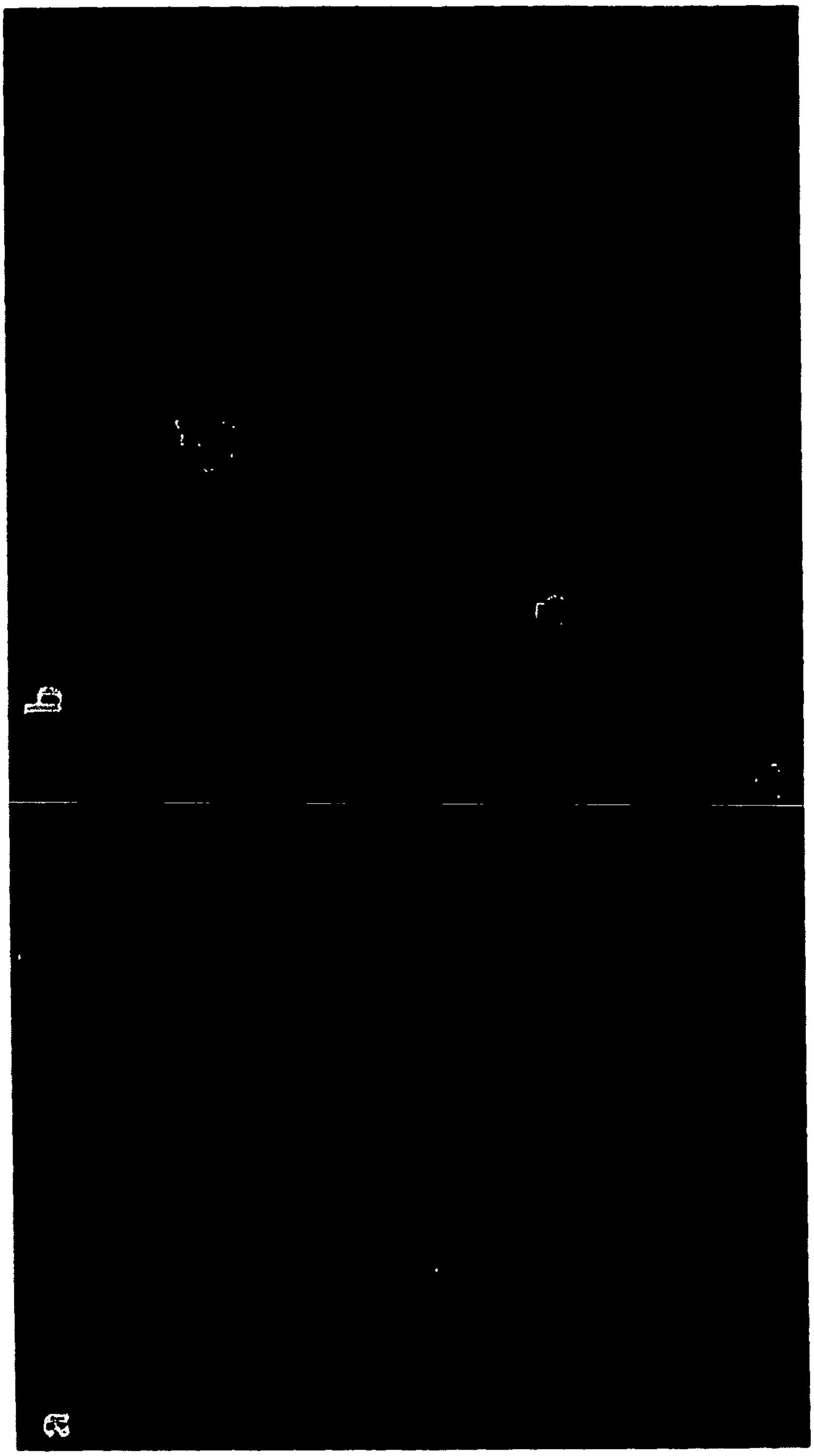

Fig. 12

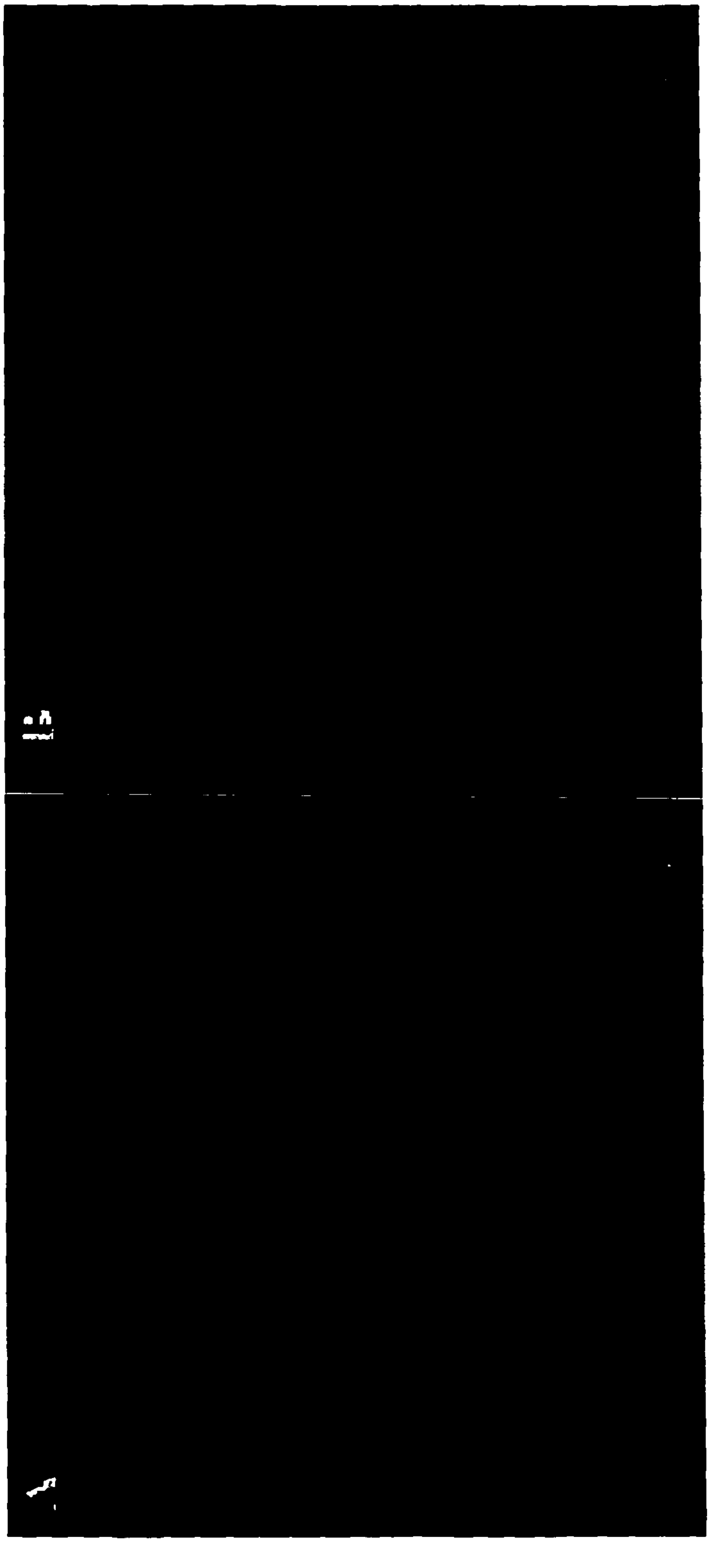

Fig. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0350990 A
- WO 9600235 A
- WO 9627658 A
- WO 9700884 A
- WO 9828323 A
- WO 9854965 A
- WO 9855498 A


### Non-patent literature cited in the description


- *Developmental Biology,* 1996, vol. 178, 393
- *J. Clin. Invest.,* 1998, vol. 102, 532
- *J. Biol. Chem.,* 1997, vol. 272, 6823-6826
- *vide Fertil. Steril55,* 1991, 1000-1004
- *Zygote,* 1995, vol. 5, 345
- *vide Mol. Reprod. Dev.,* 1996, vol. 45, 372-377
- *vide Fertil. Steril.,* 1989, vol. 52, 319-324
- *Mol. Reprod. Dev.,* 1996, vol. 45, 218-224
- *vide Theriogenology,* 2000, vol. 53, 761-771
- *vide Biol. Reprod.,* 1998, vol. 58, 1297-1302
- *vide Biol. Reprod.,* 1998, vol. 58, 558-565
- *Biol. Reprod.,* 1997, vol. 56, 1559-1564
- *Human Reproduction,* 1992, vol. 7, 49
- *vide Ann. NY Acad Sci.,* 1986, vol. 442, 251
- *Prog. Clin. Biol. Res.,* 1989, vol. 296, 273